# EUROPEAN PATENT SPECIFICATION

(11) **EP 2 539 709 B1**
(45) Date of publication and mention of the grant of the patent: **20.05.2015**
(21) Application number: 11745005.6
(22) Date of filing: 22.02.2011
(51) Int. Cl.: G01N 33/48, G01N 33/53

(54) **ALZHEIMER'S DISEASE-SPECIFIC ALTERATIONS OF PROTEIN KINASE C EPSILON (PKC-EPSILON) PROTEIN LEVELS**
MORBUS-ALZHEIMER-SPEZIFISCHE ÄNDERUNGEN VON PROTEINKINASE-C-EPSILON- (PKC-EPSILON-)PROTEINSPIEGELN
ALTÉRATIONS PROPRES À LA MALADIE D'ALZHEIMER DES NIVEAUX DE PROTÉINE KINASE C EPSILON (PKC-EPSILON)

(30) Priority: 09.07.2010 US 362893 P; 08.07.2010 US 362512 P; 22.02.2010 US 306922 P
(43) Date of publication of application: 02.01.2013
(73) Proprietor: Blanchette Rockefeller Neurosciences Institute, Morgantown, WV 26505-3409 (US)
(72) Inventor: KHAN, Tapan, K., Morgantown, WV 26505 (US); ALKON, Daniel, L., Bethesda, MD 20816 (US)
(74) Representative: Kador & Partner
(86) International application number: PCT/US2011/000315
(87) International publication number: WO 2011/102907

(56) References cited:
- US-A- 6 107 050
- US-A1- 2009 130 195
- US-A1- 2010 021 913
- US-A1- 2010 022 645
- MATSUSHIMA H ET AL: "Ca2+-dependent and Ca2+-independent protein kinase C changes in the brain of patients with Alzheimer's disease.", JOURNAL OF NEUROCHEMISTRY JUL 1996, vol. 67, no. 1, July 1996 (1996-07), pages 317-323, XP55065280, ISSN: 0022-3042
- SEUNG WOO YEON ET AL: "Blockade of PKC[epsilon] Activation Attenuates Phorbol Ester-Induced Increase of [alpha]-Secretase-Derived Secreted Form of Amyloid Precursor Protein", BIOCHEMICAL AND BIOPHYSICAL RESEARCH COMMUNICATIONS, vol. 280, no. 3, 1 January 2001 (2001-01-01), pages 782-787, XP55065278, ISSN: 0006-291X, DOI: 10.1006/bbrc.2000.4181
- AKITA Y: "Protein kinase C-epsilon (PKC-epsilon): its unique structure and function", JOURNAL OF BIOCHEMISTRY, JAPANESE BIOCHEMICAL SOCIETY / OUP, TOKYO; JP, vol. 132, no. 6, 1 December 2002 (2002-12-01), pages 847-852, XP008106992, ISSN: 0021-924X
- SAKAI ET AL.: 'Identification of PKC isoforms expressed in human bronchial smooth muscle cell.' J. SMOOTH MUSCLE RES. vol. 45, no. 1, 2009, pages 55 - 62, XP008159654
- LEE ET AL.: 'Amyloid beta peptide directly inhibits PKC activation.' MOL CELL NEUROSCI. vol. 26, no. 2, 2004, pages 222 - 231, XP003004868
- ZHU ET AL.: 'Protein kinase C epsilon suppresses Abeta production and promotes activation of alpha-secretase' BIOCHEM BIOPHYS RES COMMUN. vol. 285, no. 4, 2001, pages 997 - 1006, XP008159656
- DEACON ET AL.: 'Isoenzymes of protein kinase C: differential involvement in apoptosis and pathogenesis.' MOL PATHOL. vol. 50, no. 3, 1997, pages 124 - 131, XP008159657
- DE BARRY J ET AL: "Protein kinase C as a peripheral biomarker for Alzheimer's disease", EXPERIMENTAL GERONTOLOGY, ELSEVIER SCIENCE, OXFORD, GB, vol. 45, no. 1, 1 January 2010 (2010-01-01), pages 64-69, XP026808954, ISSN: 0531-5565 [retrieved on 2009-11-04]

## Description

### FIELD OF THE INVENTION

The present invention relates to methods of diagnosing Alzheimer's Disease by detecting alterations in the ratio of PKC epsilon protein levels in one or more peripheral cells of a human patient compared with PKC epsilon levels in one or more peripheral cells of a control subject. The Alzheimer's disease-specific molecular biomarkers disclosed herein are useful for the diagnosis of Alzheimer's disease and for screening methods for the identification of compounds for treating or preventing Alzheimer's disease. The present invention also provides methods for elevating PKC epsilon protein levels comprising the steps of contacting one or more human peripheral cells with an amount of a PKC activator effective to elevate PKC epsilon levels compared to an uncontacted human peripheral cell.

### BACKGROUND OF THE INVENTION

Alzheimer's disease (AD), the most common form of dementia, begins with the loss of recent memory and is associated with two main pathological hallmarks in the brain: extracellular amyloid plaques and intracellular neurofibrillary tangles. These are typically associated with a significant loss of synapses. Amyloid plaques are formed by the aggregation of Aβ peptide oligomers which are generated from cleavage of the amyloid precursor protein (APP) by the β- secretase and γ-secretase pathway, while a secretase generates the non-toxic, synaptogenic soluble APP-α. Accumulated observations indicate that Protein kinase C (PKC) isozymes -α and -ε directly activate the α- secretase mediated cleavage of APP directly (Slack et al., 1993; Kinouchi et al., 1995; Jolly-Tornetta and Wolf 2000; Yeon et al., 2001, Lanni et al., 2004), and/or indirectly through phosphorylation of the extracellular signal regulated kinase (ERK1/2) (Devari et al., 2006, Alkon et al., 2007). Many observations have also indicated that PKC signaling pathways regulate important events in neurodegenerative pathophysiology of AD such as the endothelin converting enzyme (ECE)-mediated degradation of Aβ (Nelson et al., 2009). In vivo over-expression of PKC-ε in AD- transgenic mice reduced amyloid plaques (Choi et al., 2006). Other studies have provided evidence that AD specific pathological abnormalities can be found in tissues other than brain which include blood, skin fibroblasts, and ocular tissues (Gurreiro et al., 2007, Ray et al., 2007). In AD skin fibroblasts, for example, defects were found of specific K⁺ channels (Etcheberrigaray et al., 1993; 1994), PKC isozymes (Govoni et al. , 1993, Favit et al., 1998), Ca⁺ signaling (Ito et al., 1994), MAP kinase Erkl/2 phosphorylation (Zhao et al., 2002; Khan and Alkon, 2006), and PP2A (Zhao et al., 2003). For familial AD patients, skin fibroblasts showed enhanced secretion of Aβ (Citron et al., 1994; Johnston et al., 1994) while AD-specific reduction of specific K+ channels was induced by AβI₋₄₀ in normal human fibroblasts (Etcheberrigaray, et al., 1993; 1994). Recently, an autopsy confirmed, internally controlled, phosphorylated Erkl/2 peripheral biomarker in skin fibroblasts was shown to have promising sensitivity and specificity (Khan and Alkon, 2006; 2010). Still other studies have suggested deficits of PKC in particular brain regions of AD patients (Masliah et al., 1991). Finally, it has also been recently demonstrated that pharmacologic activators of PKC-α and -ε can protect two different strains of AD mice from all of the pathologic and cognitive abnormalities characteristics of AD (Hongpaisan et al., 201 1).Consistent with these observations, PKC -α and -ε were found to be significantly reduced in AD transgenic mice and were restored to normal levels by treatment with pharmacologic activators of PKC-α and -ε (Hongpaisan et al., 201 1). The publication of Matsushima et al, Journal of Neurochemistry, July 1996, vol. 67, no. 1, pages 317 to 323 refers to studies regarding protein kinase C (PKC) activity in Ca²⁺-dependent PKC (Ca²⁺-dependent PKC activities) and Ca²⁺-independent PKC (Ca²⁺-independent PKC activities) assay conditions in brains from Alzheimer's disease (AD) patients and age-matched controls. Seung at al, Biochemical and Biophysical Research Communications 280, page 782 to 787, 2001, investigate the role of PKC-ε in amyloid precursor proteins (APP) processing using APP-overexpressing B103 cells. In the Journal of Biochemistry (JB) Minireview, vol. 132, pages 847 to 852, 2002, Yoshiko Akita discusses the unique structure and function PKC-ε.De Barry et al, Experimental Gerontology, vol. 45, pages 64 to 69, 2010, study the role of PKC as a peripheral biomarker for Alzheimer's disease

Collectively, these and other previous studies have two important implications: I. AD has systemic pathologic expression with symptomatic consequences limited to brain function, and II. PKC isozymes particularly -α and -ε, play a critical role in regulating the major aspects of AD pathology including the loss of synapses, the generation of Aβ and amyloid plaques, and the GSK-3β- mediated hyperphosphorylation of tau in neurofibrilliary tangles. For these reasons we analyzed the PKC-ε in skin fibroblasts from AD, age-matched controls (AC) and non-AD dementia (non-ADD) patients at the steady state levels. This report reveals that PKC-ε as well as changes in these levels induced by application of soluble Aβ oligomers may provide a diagnostic basis for AD in peripheral tissues.

There exists a need for highly sensitive and highly specific tests to diagnose Alzheimer's disease and to screen for compounds useful in the treatment and prevention of Alzheimer's disease. The present inventors have identified, for the first time, unique Alzheimer's disease-specific molecular biomarkers useful for the diagnosis of Alzheimer's disease in a highly sensitive and highly specific manner compared to previously known diagnostic tests. Thus, the unique Alzheimer's disease-specific molecular biomarkers disclosed herein serve as the basis for diagnostic methods having a high degree of sensitivity and specificity for the detection and diagnosis of Alzheimer's disease. The unique Alzheimer's disease-specific molecular biomarkers of the present invention are also useful in screening methods to identify compounds which may be used as therapeutic agents in the treatment and prevention of Alzheimer's disease. The inventors have also discovered methods for elevating PKC epsilon protein levels in peripheral cells of human patients.

### SUMMARY OF THE INVENTION

The present invention is based on the surprising finding that PKC epsilon levels in one or more peripheral cells of a human subject are lower in Alzheimer's Disease subjects (AD) than in age matched controls (AC). In certain embodiments, the invention is directed to a method of diagnosing Alzheimer's Disease , said method comprising the steps of: a) determining the PKC epsilon level in one or more peripheral cells of said human subject; and b) comparing the PKC epsilon level to the PKC epsilon level in one or more peripheral cells of a control subject; wherein said method is indicative of Alzheimer's Disease if the PKC epsilon level in one or more peripheral cells of said human subject is lower than the PKC epsilon level in one or more peripheral cells of said control subject.

In certain embodiments said PKC epsilon level is a PKC epsilon protein level or a PKC epsilon activity level. In certain embodiments, the PKC epsilon level is measured by RT-PCR. In certain embodiments, the control subject does not have Alzheimer's Disease. The diagnostice methods of the present invention are conducted *in vitro.*

In certain preferred embodiments of the invention, said one or more peripheral cells is a fibroblast, buccal mucosal, neuron, or blood cell.

In certain embodiments, the measuring or determining steps of the level of PKC epsilon in one or more peripheral cells steps comprises a method selected from the group consisting of radioimmunoassay, Western blot assay, immunofluorescent assay, enzyme immunoassay, immuno-precipitation, chemiluminescent assay, immunohistochemical assay, dot blot assay and slot blot assay.

In certain embodiments, the absence of Alzheimer's Disease in said human subject is indicated if said PKC epsilon level in one or more peripheral cells of said human subject is greater than or equal to the PKC epsilon level in one or more peripheral cells of said control subject.

Furthermore, the invention is directed to a method of diagnosing Alzheimer's Disease comprising the steps of: a) determining the PKC epsilon level in - one or more peripheral cells of a human subject b) contacting said one or more cells of step (a) with an agent that is a PKC epsilon activator; c) determining the PKC epsilon level in said one or more cells in step (b) after said contacting in step (b); wherein Alzheimer's Disease is indicated in said human subject if the PKC epsilon level determined in step (c) is greater that the PKC epsilon level determined in step (a).

In certain embodiments, the absence of Alzheimer's Disease in said human subject is indicated if said PKC epsilon level in determined in step (c) is equal to or less than the PKC epsilon level determined in step (a).

In certain embodiments, the invention is directed to a method of determining or monitoring Alzheimer's Disease progression comprising the steps of: a) determining the PKC epsilon level in one or more peripheral cells of said human subject; b) comparing the PKC epsilon level in the one or more peripheral cells of said human subject to the PKC epsilon level in one or more peripheral cells of a control subject; and c) determining or monitoring said Alzheimer's Disease progression based on said comparison in step (b).

In certain embodiments, the PKC epsilon level in one or more peripheral cells of said human subject decreases as Alzheimer's Disease progresses over time.

In certain embodiments, the PKC epsilon level increases in one or more peripheral cells of said human subject as Alzheimer's Disease progression is reversed.

In certain preferred embodiments, the invention is directed to methods for elevating the PKC epsilon protein level in a cell, comprising the step of contacting one or more human cells with an amount of a PKC activator effective to elevate the PKC epsilon protein level in said cell compared to an uncontacted human cell.

In certain embodiments, said human peripheral cell is a fibroblast, buccal mucosal, neuron, or blood cell. In certain embodiments said PKC activator is a macrocyclic lactone. In certain embodiments, said macrocyclic lactone is a bryostatin. In certain embodiments, said bryostatin is bryostatin-1. In certain embodiments, said PKC epsilon level is a PKC epsilon protein level or a PKC epsilon activity level.

Additionally, the invention is directed to methods of diagnosing Alzheimer's Disease comprising the steps of: a) determining the PKC epsilon level in one or more peripheral cells of a human subject; b) contacting said one or more peripheral cells of step (a) with an Aβ peptide; c) determining the PKC epsilon level in said one or more peripheral cells in step (b) after said contacting in step (b); wherein Alzheimer's Disease is indicated if the PKC epsilon level determined in step (c) is not significantly different from the PKC epsilon level determined in step (a).

In certain embodiments, the absence of Alzheimer's Disease is indicated if said PKC epsilon level in determined in step (c) is less than the PKC epsilon level determined in step (a).

In certain embodiments, the invention is directed to kits comprising one or more antibodies specific for PKC epsilon. In certain embodiments said kit may comprise a PKC activator. In certain embodiments, said kit may comprise a PKC epsilon activator. In certain embodiments, said kit may comprise one or more oligonucleotides specific for a gene encoding PKC epsilon.

In certain embodiments, the invention is directed to a kit comprising one or more oligonucleotides specific for a gene encoding PKC epsilon. In certain embodiments, said kit may comprise a PKC activator. In certain embodiments, said kit may comprise a PKC epsilon activator.

In certain embodiments, the invention is directed to a method of identifying a compound useful for the treatment of Alzheimer's Disease comprising: a) determining the PKC epsilon level in one or more peripheral cells of an Alzheimer's Disease subject; b) contacting said peripheral cells with a candidate compound; c) determining the PKC epsilon level in said one or more peripheral cells after said contacting step (b); wherein said candidate compound is identified as a compound useful for the treatment of Alzheimer's Disease if the PKC epsilon level determined in step (c) is greater than the PKC epsilon level determined in step (a).

Protein kinase C (PKC) isozymes particularly -α and -ε, play a critical role in regulating major aspects of AD pathology including the loss of synapses, the generation of Aβ and amyloid plaques, and the GSK-3β -mediated hyperphosphorylation of tau in neurofibrilliary tangles. Evidence of AD-specific signaling deficits has been previously found in peripheral tissues such as blood, skin fibroblasts, and ocular tissues. PKC-ε is an accurate AD Biomarker in AD skin fibroblasts.

In certain embodiments, basal protein levels of PKC-ε may be measured by western blot, immuno- fluorescence and at the transcript level by RT-PCR in cultured skin fibroblasts of AD patients, age-matched control (AC) cases, and non-AD dementia patients. Eleven AC, and ten AD subjects are selected both from sporadic and familial cases with the presence of amyloid plaques and neurofibrillary tangles in brain at autopsy (9 autopsy confirmed out of 10 AD cases). Eight inherited Huntington's disease (HD) patients with genetic evidence of non-AD characteristics, one Parkinson's disease patient, and one fronto-temporal dementia patient are included to establish that the deficiency of PKC-ε is due to only AD pathology.

PKC-ε levels in all the AD fibroblasts are found lower than the AC and non-AD dementia fibroblasts. The average PKC-ε in AD (0.501 ± 0.021, A.U.) is found ∼40% lower than the AC (0.857 ± 0.036, A.U.), and much lower than non-AD dementia (1.040 ± 0.288, A.U.) cases in western blots when normalized with respect to beta tubulin. A similar change is also found after immunofluorescence analysis. The mRNA level of PKC-ε (AC: 0.904 ± 0.103, AD: 0.530 ± 0.061) is also found to be lowered than that of AD patients. After oligomeric Aβ application to skin fibroblasts, the PKC-ε levels decreases in fibroblasts from AC, but not AD patients, indicating a pathophysiological Aβ effect on PKC-ε.

The inventors find, that PKC-ε levels are significantly lowered in the AD cultured skin fibroblasts compared to healthy AC and non-AD dementia cases. PKC-ε is a peripheral diagnostic biomarker and a therapeutic target for AD.

### BRIEF DESCRIPTION OF THE FIGURES

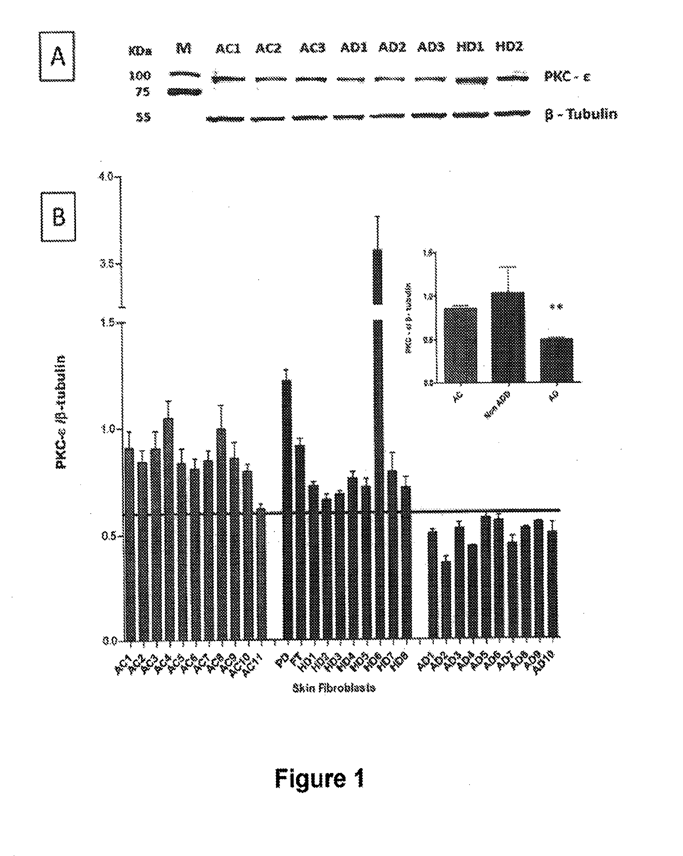

Figure 1A and IB: PKC-ε expression in cultured human fibroblasts from age-matched controls (AC), Alzheimer's disease (AD), and non-AD dementia. Values are means ± SEM of three independent experiments.
Figure 1A: Immunoreactivity of PKC-ε and β-tubulin in AC, AD, and non-AD dementia fibroblasts. AC1, AC2, and AC3 (AG07714, AG1 1734 and AG 12927) are age matched control fibroblasts, AD1, AD2 and AD3 (AG06844, AG04159 and AG08245) are Alzheimer's disease fibroblasts and HD1 and HD2 (GM06274, GM04198) are Huntington's disease fibroblasts, respectively.
Figure 1B: Graphical representation of normalized densitometric ratios of PKC-ε to β-tubulin in eleven AC, ten AD, eight HD, one Parkinson's disease (PD) and one Frontotemporal dementia (FT). In AC cells the ratios varied between 0.7 - 1.2 (Y-axis), in the non-AD dementia the ratios varied between 0.72 - 1.3 (with two exceptions HD6 and HD8) while in AD the ratios of all the cell lines were below 0.6. Inset in Panel B: Mean values were 0.857 ± 0.0361 (SEM) in AC cells, 1.040 ± 0.288 in non-AD dementia and 0.501 ± 0.021 in AD cells. PKC-ε was significantly lower in AD compared to AC (p<0.0001) and non-AD dementia (p= 0.0394). The mean of AC1 1 (0.6213±0.040) was the lowest among the ACs. However, it was also significantly different (P=0.0162) when compared with all AD cases.

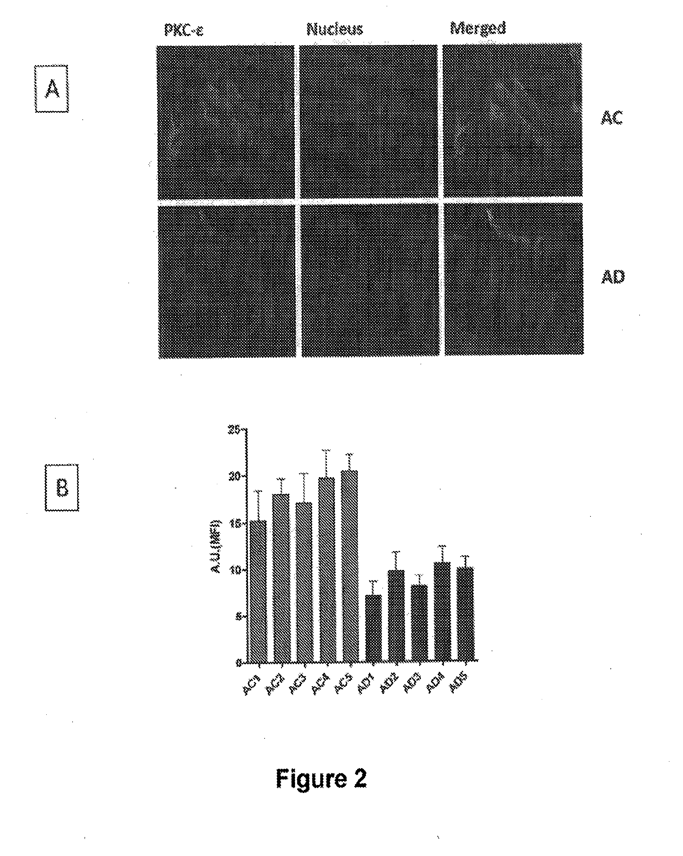

Figure 2A and 2B: Immuno fluorescence detection of PKC-ε in cultured human fibroblasts from age matched control (AC) and Alzheimer's disease (AD) fibroblasts.
Figure 2A: Confocal micrographs of age-matched control (AC), and Alzheimer's disease fibroblasts (AD). Green channel (FITC) represents PKC-ε, the blue channel is (DAPI) nuclear stain indicator, and the third channel represents a merged image. The Mean fluorescence intensity (MFI) from green (for PKC-ε) was measured from all cells for 5 different fields from each of five AC's and AD's fibroblasts. Values are mean ± SEM.
Figure 2B: Graphical representation of MFI (mean fluorescence intensity) of PKC-ε from 5 AC cases (AG07714, AG11734, AG05840, AG06242 and AG12927) and 5 AD cases (AG06844, AG04159, AG06840, AG05770 and AG08245. In AC cells the MFI varied between 15- 20A.U. (Y-axis), while in AD the range is in between 7-10. The average intensity of PKC-ε in AC's and AD were 18.092 ± 2.087 and 9.110 ± 1.420, respectively.

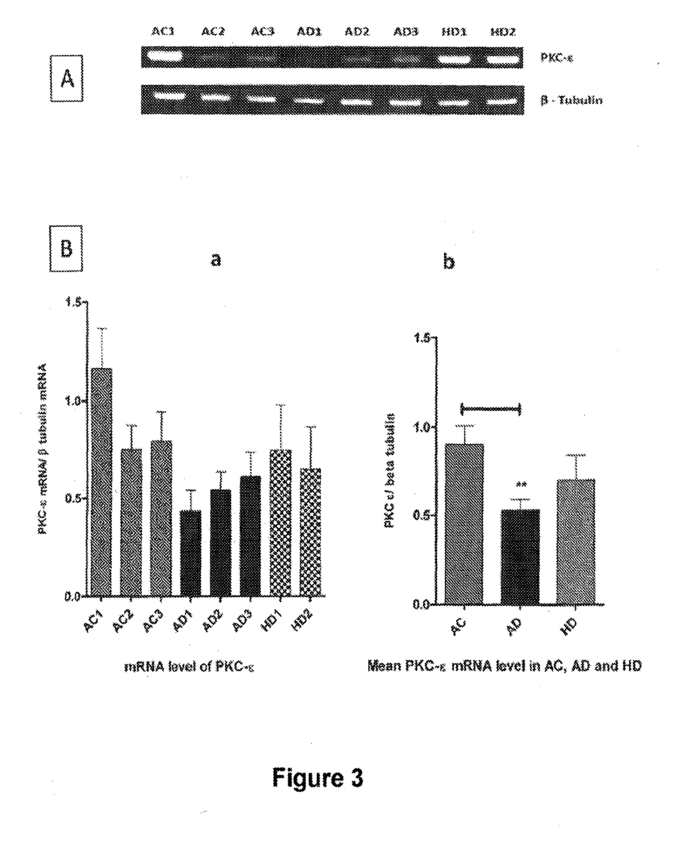

Figure 3A and 3B: RT-PCR analysis of PKC-ε.
Figure 3A: mRNA was isolated from three AC, three AD and two HD's cases. RT-PCR amplicons of PKC-ε, and β-tubulin were run on E-Gels and imaged on a Fuji gel scanner. (AC1, AC2 and AC3: AG1 1363, AG09977 and AG12998, respectively; AD1, AD2 and AD3: AG06263, AG10788 and AG08259, respectively; HD1 and HD2:GM02165 and GM04226, respectively).
Figure 3B: (a). Histogram representing the normalized value of PKC ε with respect to β-tubulin for three AC's, three AD's and two HD's. Values represent mean ± SEM of three independent experiments, (b) The mean PKC-ε mRNA level of the AD cells were significantly (p< 0.0033) lower than the AC cells (AC: 0.904 ± 0.103, AD: 0.530 ± 0.061 and HD: 0.701 ± 0.143).

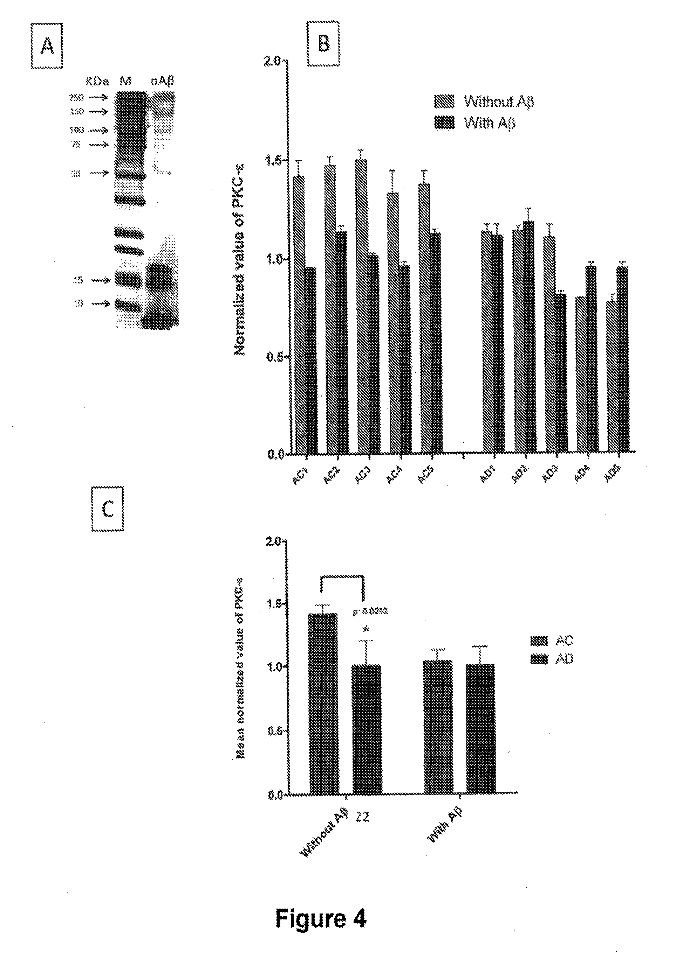

Figure 4A, 4B and 4C: Soluble Aβ oligomers induce Alzheimer's PKC-ε phenotype of human fibroblasts.
Figure 4A: SDS - PAGE analysis of the synthesized Aβ oligomers from Aβ_{I-42.} Lane M: Protein molecular weight marker, Lane oAβ: Soluble Aβ oligomers.
Figure 4B: Soluble Aβ oligomer (500nM) treatment decreases the PKC-ε in all age-matched control skin fibroblasts (five AD and five AC cases). Mean normalized densitometric values of PKC-ε were calculated from five different cell lines (Aβ oligomers treated and untreated skin fibroblasts). In each case the AC value was calculated considering the AD mean value as one. ACs showed significant decrease in PKC-ε expression after Aβ treatment, (p values are 0.0044, 0.0035, 0.0005, 0.0330 and 0.0253 for AC1, AC2, AC3, AC4 and AC5, respectively), while AD cases did not show decrease in expression.
Figure 4C: Aβ oligomer treatment changed the AC to an AD phenotype. PKC-ε levels showed no significant difference in Aβ oligomer treated AC and AD cells, while in untreated cells AD showed a 40% reduced expression compared to AC (P=0.0292).
Figure 5: Interaction of PKC-ε with Aβ, implication in Alzheimer's disease. In AD pathology the over production Aβ by higher β-,γ- sectretase activity and lower a-secretase activity decrease the amount of PKC-ε. On the other hand PKC- α and PKC-ε increase the α-secretase activity, PKC-ε also increases the activity of Aβ degrading enzymes, particularly ECE (endothelin converting enzyme).
Figure 6: Structures of molecules contemplated for use according to the present invention (BR-101 through BR-1 18).

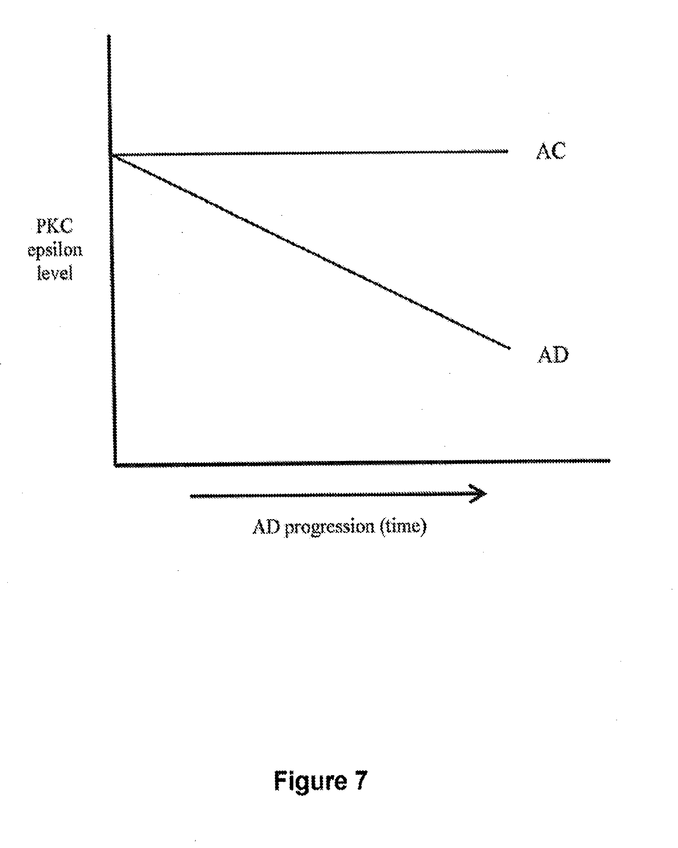

Figure 7: Schematic diagram of reduction of PKC epsilon level over time as a function of Alzheimer's Disease progression; or severity of cognitive impairment; or disease duration. PKC epsilon level may be an activity level, the protein level in one or more cells or transcript level measured, for example, by RT-PCR.

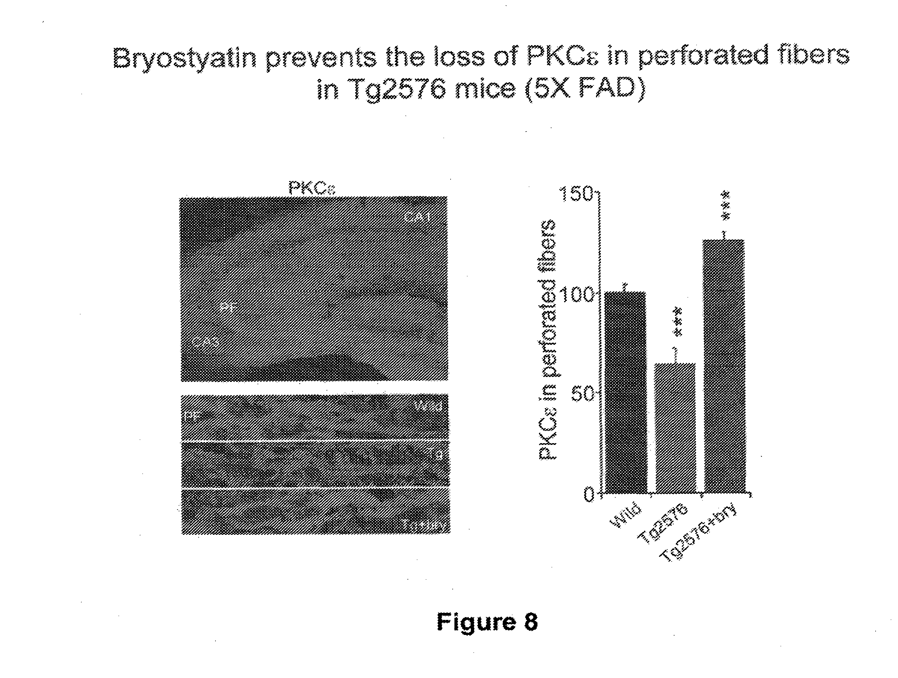

Figure 8: Bryostatin prevents the loss of PKCε in perforated fibers in Tg2576 mice (5x FAD)

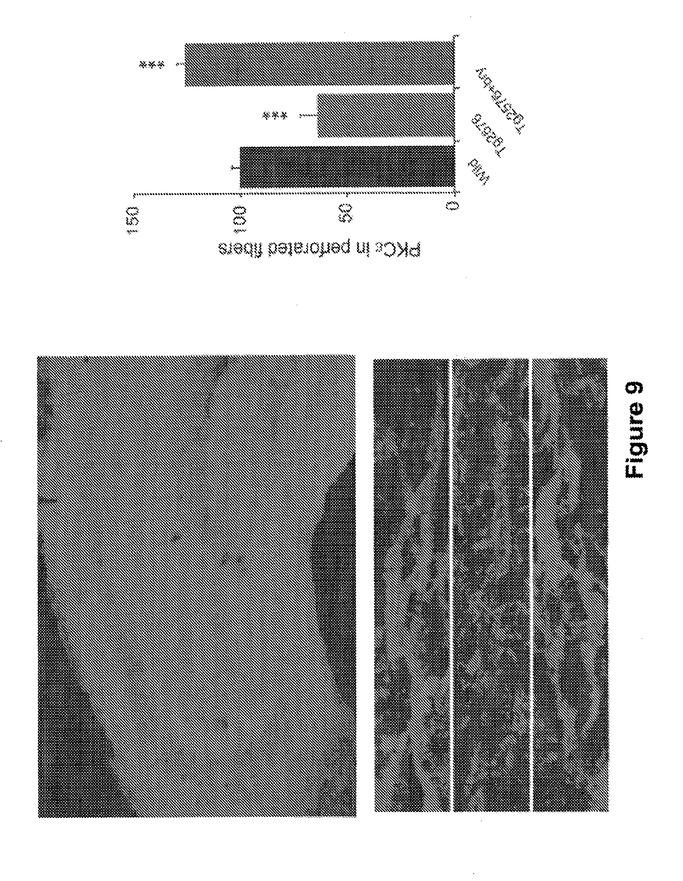

Figure 9: PKCε in perforated fibers with and without bryostatin.

### DETAILED DESCRIPTION OF THE INVENTION

As used herein, the term "PKC epsilon level" includes, but is not limited to, any one or more of the following: the enzymatic activity of PKC epsilon, the amount of PKC epsilon protein, or the amount of RNA encoding PKC epsilon.

A "fatty acid" is a carboxylic acid with an unbranched aliphatic chain containing from about 4 to 30 carbons; most long chain fatty acids contain between 10 and 24 carbons. Fatty acids can be saturated or unsaturated. Saturated fatty acids do not contain any double bonds or other functional groups along the chain. Unsaturated fatty acids contain one or more alkenyl functional groups, i.e., double bonds, along the chain. The term "polyunsaturated fatty acid" or "PUFA" means a fatty acid containing more than one double bond. There are three classes of PUFAs, omega-3 PUFAs, omega- 6 PUFAs, and omega-9 PUFAS. In omega-3 PUFAs, the first double bond is found 3 carbons away from the last carbon in the chain (the omega carbon). In omega-6 PUFAs the first double bond is found 6 carbons away from the chain and in omega-9 PUFAs the first double bond is 9 carbons from the omega carbon.

PUFAs are also called "polyenoic fatty acids." As used herein, the term PUFA includes both naturally-occurring and synthetic fatty acids. A major source for PUFAs is from marine fish and vegetable oils derived from oil seed crops, although the PUFAs found in commercially developed plant oils are typically limited to linoleic acid and linolenic acid (18:3 delta 9,12,15).

A "cis-PUFA" is one in which the adjacent carbon atoms are on the same side of the double bond.

The abbreviation X:Y indicates an acyl group containing X carbon atoms and Y double bonds. For example, linoleic acid would be abbreviated 18:2.

A "methylene-interrupted polyene" refers to a PUFA having two or more *cis* double bonds separated from each other by a single methylene group.

A "non-methylene-interrupted polyene," or "polymethylene- interrupted fatty acid," refers to a PUFA having two or more cis double bonds separated by more than one methylene group.

A "monounsaturated fatty acid" (MUFA) is a fatty acid that has a single double bond in the fatty acid chain and all the remaining carbon atoms in the chain are single-bonded. Exemplary MUFAs include oleic acid, myristoleic acid and palmitoleic acid.

A "cis-monounsaturated fatty acid" means that adjacent hydrogen atoms are on the same side of the double bond.

Conjugated fatty acids such as conjugated linoleic acid {9-*cis*,11-trans-octadecadienoic acid) possess a conjugated diene, that is, two double bonds on adjacent carbons. Some evidence suggests that conjugated linoleic acid has antitumor activity.

Exemplary PUFAs include lineoleic acid (9,12-octadecadienoic acid); γ-linolenic acid (GLA; 6,9,12-octadecatrienoic acid); a-linolenic acid (9,12,15-octadecatrienoic acid); arachidonic acid (5,8,1 1,14-eicosatetraenoic acid); eicosapentanoic acid (EPA; 5,8,1 1,14,17-eicosapentanoic acid); docosapentaenoic acid (DPA; 7,10,13,16,19-docosapentaenoic acid); docosahexaenoic acid (DHA; 4,7,10,13,16,19-docosahexanoic acid); and stearidonic acid (6,9,12,15-octadecatetraenoic acid).

As used herein, the term "cyclopropane" refers to a cycloalkane molecule with the molecular formula C3H6, consisting of three carbon atoms linked to each other to form a ring, with each carbon atom bearing two hydrogen atoms.

An "epoxide" refers to a cyclic ether with three ring atoms.

As used herein, a "PUFA derivative" refers to a PUFA, or alcohol or ester thereof, in which at least one of the double bonds has been cyclopropanated or epoxidized.

As used herein, a "MUFA derivative" refers to a MUFA, or alcohol or ester thereof, in which the double bond has been cyclopropanated or epoxidized.

"Selective activation" of PKCε means that the PUFA derivative compound of the present invention activates PKCs to a greater detectable extent than any other PKC isozyme. In specific embodiments, the PUFA derivative activates PKCε at least 1-fold, 2-fold or 5 -fold over the other PKC isozymes as measured using e.g., the PKC activation assay described herein. Upon activation, protein kinase C enzymes are translocated to the plasma membrane by RACK proteins (membrane-bound receptor for activated protein kinase C proteins). In general, upon activation, protein kinase C enzymes are translocated to the plasma membrane by RACK proteins. Other indications of PKC activation include phosphorylation at specific C-terminal serine/threonine residues by phosphatidylinositol-trisphosphate-dependent kinase (PDK1), with at least two additional phosphorylations and/or autophosphorylations of well-conserved sequences in each enzyme of the PKC family. Activation of PKC is described in Sun and Alkon, Recent Patents CNS Drug Discov. 2006; 1(2): 147-56.

"Neurodegeneration" refers to the progressive loss of structure or function of neurons, including death of neurons.

For purposes of the present invention, a "neurological disease" refers to any central nervous system (CNS) or peripheral nervous system (PNS) disease that is associated with the β-amyloidogenic processing of APP. This may result in neuronal or glial cell defects including but not limited to neuronal loss, neuronal degeneration, neuronal demyelination, gliosis (i.e., astrogliosis), or neuronal or extraneuronal accumulation of aberrant proteins or toxins (e.g., Aβ).

One exemplary neurological disease is Alzheimer's Disease (AD).

Another exemplary neurological disease is congophilic angiopathy (CAA), also referred to as cerebral amyloid angiopathy.

The term "Alzheimer's Disease" or "AD" refers to any condition where Aβ deposition will eventually accumulate in the cells of the central nervous system. In one, non-limiting embodiment, Aβ, particularly Aβ1-42, peptide is formed from the β-amyloidogenic metabolism of APP. AD may be heritable in a Familial manifestation, or may be sporadic. Herein, AD includes Familial, Sporadic, as well as intermediates and subgroups thereof based on phenotypic manifestations.

Another neurological disease is Down syndrome (DS). Subjects with DS invariably develop (in their third or fourth decade) cerebral amyloid (Aβ) plaques and neurofibrillary tangles (NFTs), the characteristic lesions of AD. Recent studies have shown that the Aβ42 is the earliest form of this protein deposited in Down syndrome brains, and may be seen in subjects as young as 12 years of age, and that soluble Aβ can be detected in the brains of DS subjects as early as 21 gestational weeks of age, well preceding the formation of Aβ plaques. Gyure et al., Archives of Pathology and Laboratory Medicine. 2000; 125:. 489-92.

As used herein, the term "subject" includes a mammal.

The phrase "pharmaceutically acceptable" refers to molecular entities and compositions that are physiologically tolerable and do not typically produce untoward reactions when administered to a subject. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans. The term "pharmaceutically acceptable carrier" means a chemical composition with which the active ingredient may be combined and which, following the combination, can be used to administer the active ingredient to a subject and can refer to a diluent, adjuvant, excipient, or vehicle with which the compound is administered.

The terms "therapeutically effective dose" and "effective amount" refer to an amount of a therapeutic agent that results in a measurable therapeutic response. A therapeutic response may be any response that a user (*e.g*., a clinician) will recognize as an effective response to the therapy, including improvement of symptoms and surrogate clinical markers. Thus, a therapeutic response will generally be an amelioration or inhibition of one or more symptoms of a disease or condition *e.g.,* AD. A measurable therapeutic response also includes a finding that a symptom or disease is prevented or has a delayed onset, or is otherwise attenuated by the therapeutic agent.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values.

Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

### PKC-ε levels are lower in AD fibroblasts

In this study population we have included six sporadic (late onset, without family history,) and four familial (early onset) cases of AD to test that PKC-ε is dysfunctional in both sporadic and familial cases and is a hallmark of AD pathological signaling. Immunoblot analysis of 10 AD cell lines, 11 age AC cell lines and 10 non-AD dementia fibroblasts revealed that the PKC-ε levels in the AD samples were lower by approximately 40% compared to the AC (Fig. 1). The average normalized ratio of PKC-ε against β-tubulin was 0.857 ± 0.036 (SEM) in AC cases (n=11), 1.040 ± 0.288 in non-AD dementia (n=10), and 0.501 ± 0.021 in AD cells (n=10). The PKC-ε levels were significantly lower in the AD fibroblasts (p<0.0001) compare to AC cases. The mean basal PKC-ε level for the AC 11 case (0.6213±0.040) was the lowest among all ACs (Figure 1B). However, the basal level of PKC-ε of AC1 1 was also statistically significant when compared separately with all AD cases (P=0.0162).

Data from western blot analysis were supported by immunofluorescence analysis of stained fibroblasts that demonstrated distinct differences in intensity of PKC-ε tagged with FITC between AC and AD cells (Fig. 2). The average intensity of PKC-ε in AC and AD cells were 18.092 ± 2.087 and 9.110 ± 1.420, respectively. To test for dysfunctional PKC-ε at the transcript level, RT-PCR experiments were conducted. The average mRNA levels of three AD, three AC and two HD cell lines were measured (Fig. 3). All PKC-ε mRNA levels were normalized with β- tubulin mRNA levels of corresponding cell lines. The normalized average mRNA level of AD cells were significantly (p< 0.003) lower than the AC and HD cells (AC: 0.904 ± 0.103, AD: 0.530 ± 0.061 and HD: 0.701 ± 0.143) (Fig. 3).

### Treatment of skin fibroblasts with oligomeric Aβ

Oligomeric Aβ synthesized by the method described earlier produced high molecular weight oligomers with molecular weight of > 100kDa size (Fig. 4A). These oligomers were reported to be highly toxic and had similarities to those found in the AD brain (Nouguchi et al., 2009). To establish the pathophysiological relevance of AD, exogenous toxic oligomeric Aβ₁₋₄₂ was added to the normal fibroblasts (AC) and the impact on PKC-ε expression level was assessed at basal state. After treatment with oligomeric Aβ, the PKC-ε levels were found decreased in the AC cases, while the AD cases showed no statistical difference. Average of the mean of three independent experiments from five different AC and AD patients was calculated following oligomeric Aβ treatment and was compared to the untreated cells. Untreated AC and AD cells showed a difference of ∼40% among them, while treated AC and AD cells demonstrated no difference in PKC-ε expression or were sometimes higher for the AD cases after Aβ treatment.

The present invention relates, in certain aspects, to methods of diagnosing Alzheimer's disease in human peripheral cells taken from subjects that have been identified for testing and diagnosis. The diagnosis is based upon the discovery of unique Alzheimer's disease-specific molecular biomarkers. In certain aspects, the invention is directed to methods of monitoring Alzheimer's disease progression and to screening methods for the identification of lead compounds for treating or preventing Alzheimer's disease.

Because direct access to neurons in the brains of living human beings is impossible, early diagnosis of Alzheimer's disease is extremely difficult. By measuring the Alzheimer's disease-specific molecular biomarkers disclosed herein, the present invention provides highly practical, highly specific and highly selective tests for early diagnosis of Alzheimer's disease. In addition, the Alzheimer's disease-specific molecular biomarkers described herein provide a basis for following disease progression and for identifying therapeutic agents for drug development targeted to the treatment and prevention of Alzheimer's disease.

The inventors have found a unique molecular biomarker for Alzheimer's disease using peripheral (non-CNS) tissue that is useful in diagnostic assays that are highly sensitive and highly specific for the diagnosis of Alzheimer's disease. A great advantage is that the tissue used in the assays and methods disclosed herein may be obtained from subjects using minimally invasive procedures, i.e., without the use of a spinal tap. Thus, one aspect of the invention is directed to an assay or test for the early detection of Alzheimer's disease.

The invention is also directed to methods for screening a test compound (or a lead compound) useful for the treatment or prevention of Alzheimer's disease wherein the methods comprise an in vitro assay.

In further embodiments of the invention, the protein kinase C activator is selected from the group consisting of bradykinin, bryostatin, bombesin, cholecystokinin, thrombin, prostaglandin F2 alpha and vasopressin. The cells are peripheral cells. In still further embodiments of the invention, the peripheral cells are selected from the group consisting of skin cells, skin fibroblast cells, blood cells and buccal mucosa cells. In still further embodiments of the invention, the cells are not isolated from cerebral spinal fluid. In still further embodiments of the invention, the cells do not comprise cerebral spinal fluid. In still further embodiments of the invention, the cells are not obtained by a spinal tap or lumbar puncture. In still further embodiments of the invention, the protein kinase C activator is contacted with said cells in media comprising serum. In still further embodiments of the invention, the protein kinase C activator is contacted with said cells in serum-free media. In still further embodiments of the invention, the PKC epsilon proteins are detected by immunoassay. In still further embodiments of the invention, the immunoassay is a radioimmunoassay, a Western blot assay, an immunofluoresence assay, an enzyme immunoassay, an immunoprecipitation assay, a chemiluminescence assay, an immunohistochemical assay, an immunoelectrophoresis assay, a dot blot assay, or a slot blot assay. In still further embodiments of the invention, the measuring is done using a protein array, a peptide array, or a protein micro array.

In further embodiments of the invention, the PKC activator, or pharmaceutical composition, comprises any of the following compounds selected from the group consisting of DCP-LA; DCPLA methyl ester, DHA-CP6 methyl ester (BR-111); EPA-CP5 methyl ester (BR-1 14); AA-CP4 methyl ester (BR-1 15); DHA-CP6;
EPA-CP5; AA-CP4; Linolenyl alcohol cyclpropanated (BR- 104); Linoleic alcohol cyclopropanated (BR- 105); Elaidic alcohol cyclopropanated (BR- 106); Elaidic acid cyclopropanated (BR-107); Oleyl alcohol cyclopropanated(BR-108); Vernolic acid cyclopropanated methyl ester (BR-109); Linolenic acid cyclopropanated (BR-1 18); Elaidic acid cyclopropanated methyl ester; Vernolic acid cyclopropanated; Linolenic acid cyclopropanated methyl ester;
8-(2-((2-pentylcyclopropyl)methyl)cyclopropyl)octanoicacid (DCP-LA);
methyl 3-(2-((2-((2-((2-((2-((2-ethylcyclopropyl)methyl)cyclopropyl)methyl)cyclopropyl)methyl)-cyclopropyl)methyl)cyclopropyl)methyl)cyclopropyl)propanoate
methyl 3-(2-((2-((2-((2-((2-((2-ethylcyclopropyl)methyl)cyclopropyl)methyl)cyclopropyl)methyl)-cyclopropyl)methyl)cyclopropyl)methyl)cyclopropyl)propanoate (DHA-CP6 methyl ester);
methyl 4-(2-((2-((2-((2-((2-ethylcyclopropyl)methyl)cyclopropyl)methyl)cyclopropyl)-methyl)cyclopropyl)methyl)cyclopropyl)butanoate
methyl 4-(2-((2-((2-((2-((2-ethylcyclopropyl)methyl)cyclopropyl)methyl)cyclopropyl)-methyl)cyclopropyl)methyl)cyclopropyl)butanoate (EPA-CP5 methyl ester)
methyl 4-(2-((2-((2-((2- pentylcyclopropyl)methyl)cyclopropyl)methyl)-cyclopropyl)methyl)cyclopropyl)butanoate
methyl 4-(2-((2-((2-((2- pentylcyclopropyl)methyl)cyclopropyl)methyl)-cyclopropyl)methyl)cyclopropyl)butanoate (AA-CP4 methyl ester)

In the methods of the invention, the cells are preferably skin fibroblasts, but any other peripheral tissue cell (i.e. outside of the central nervous system) may be used in the tests of this invention if such peripheral cells are more convenient to obtain or process. Other suitable cells include, but are not limited to, blood cells such as erythrocytes and lymphocytes, buccal mucosal cells, nerve cells such as olfactory neurons, cerebrospinal fluid, urine and any other peripheral cell type. In addition, the cells used for purposes of comparison do not necessarily have to be from healthy donors.

The peripheral cells may be fresh or may be cultured (see, U.S. Patent No. 6,107,050). In a specific embodiment, a punch skin biopsy can be used to obtain skin fibroblasts from a subject. These fibroblasts are analyzed directly using the techniques described herein or introduced into cell culture conditions. The resulting cultured fibroblasts are then analyzed as described in the examples and throughout the specification. Other steps may be required to prepare other types of cells which might be used for analysis such as buccal mucosal cells, nerve cells such as olfactory cells, blood cells such as erythrocytes and lymphocytes, etc. For example, blood cells can be easily obtained by drawing blood from peripheral veins. Cells can then be separated by standard procedures (e.g. using a cell sorter, centrifugation, etc.) and later analyzed.

Thus, the present invention relates, in certain aspects, to methods for the diagnosis and treatment of Alzheimer's disease in a subject. The invention is also directed, in certain embodiments, to kits containing reagents useful for the detection or diagnosis of Alzheimer's disease. In certain aspects, the invention is directed to methods for screening to identify lead compounds useful for treating Alzheimer's disease as well as to methods of using these compounds or chemical derivatives of the lead compounds in pharmaceutical formulations to treat or prevent Alzheimer's disease in subjects in need thereof.

Protein kinase C activators that are specifically contemplated for use in the diagnostic methods, kits and methods of screening to identify compounds of the instant invention include, but are not limited to: Bradykinin; ε-APP modulator; Bryostatin 1 ; Bryostatin 2; DHI; 1,2-Dioctanoyl-sn-glycerol; FTT; Gnidimacrin, Stellera chamaejasme L.; (-)-Indolactam V; Lipoxin A4; Lyngbyatoxin A, Micromonospora sp.; Oleic acid; I-Oleoyl-2-acetyl-sn-glycerol; 4a-Phorbol; Phorbol-12, 13-dibutyrate; Phorbol- 12, 13-didecanoate; 4α-Phorbol- 12, 13-didecanoate; Phorbol- 12-myristate- 13- acetate; L-α-Phosphatidylinositol-3,4-bisphosphate, Dipalmitoyl-, Pentaammonium Salt; L-α -Phosphatidylinositol-4,5-bisphosphate, Dipalmitoyl-, Pentaammonium Salt; L-a - Phosphatidylinositol-3,4,5-trisphosphate, Dipalmitoyl-, Heptaammonium Salt; 1- Stearoyl-2-arachidonoyl-sn-glycerol; Thymeleatoxin, Thymelea hirsuta L.; insulin, phorbol esters, lysophosphatidylcholine, lipopolysaccharide, anthracycline dannorubicin and vanadyl sulfate. Also included are compounds known as "bryologues." Bryologues are described, for example, in Wender et al. Organic letters ( United States ) May 12, 2005 ,7 (10) p1995-8; Wender et al. Organic letters ( United States ) Mar 17 2005 , 7 (6) p1177-80; Wender et al. Journal of Medicinal Chemistry ( United States ) Dec 16 2004, 47 (26) p6638-44. A protein kinase C activator may be used alone or in combination with any other protein kinase C activator in the diagnostic methods, kits and methods of screening compounds disclosed herein.

Bradykinin is a potent vasoactive nonapeptide that is generated in the course of various inflammatory conditions. Bradykinin binds to and activates specific cell membrane bradykinin receptor(s), thereby triggering a cascade of intracellular events leading to the phosphorylation of proteins known as "mitogen activated protein kinase" (MAPK). Phosphorylation of protein, the addition of a phosphate group to a Ser, Thr, or Tyr residue, is mediated by a large number of enzymes known collectively as protein kinases. Phosphorylation normally modifies the function of, and usually activates, a protein. Homeostasis requires that phosphorylation be a transient process, which is reversed by phosphatase enzymes that dephosphorylate the substrate. Any aberration in phosphorylation or dephosphorylation may disrupt biochemical pathways and cellular functions. Such

Immunoassays of the present invention for the detection of proteins may be immunofluorescent assays, radioimmunoassays, Western blot assays, enzyme immunoassay, immuno-precipitation, chemiluminescent assay, immunohistochemical assay, dot or slot blot assay and the like. (In "Principles and Practice of Immunoassay" (1991) Christopher P. Price and David J. Neoman (eds), Stockton Press, New York, New York, Ausubel et al. (eds ) (1987) in "Current Protocols in Molecular Biology" John Wiley and Sons, New York, New York). Detection may be by colorometric or radioactive methods or any other conventional methods known to those having skill in the art. Standard techniques known in the art for ELISA are described in Methods in Immunodiagnosis, 2nd Edition, Rose and Bigazzi, eds., John Wiley and Sons, New York 1980 and Campbell et al., Methods of Immunology, W.A. Benjamin, Inc., 1964, both of which are incorporated herein by reference. Such assays may be direct, indirect, competitive, or noncompetitive immunoassays as described in the art (In "Principles and Practice of Immunoassay" (1991) Christopher P. Price and David J. Neoman (eds), Stockton Pres, NY, NY; Oellirich, M. 1984. J. Clin. Chem. Clin. Biochem. 22: 895-904 Ausubel, et al. (eds) 1987 in Current Protocols in Molecular Biology, John Wiley and Sons, New York, New York.

As stated previously, the peripheral cells taken from the patient being diagnosed may be any peripheral cell. Examples of peripheral cells that may be used include, but are not limited to, skin cells, skin fibroblasts, buccal mucosal cells, blood cells, such as erythrocytes, lymphocytes and lymphoblastoid cells, and nerve cells and any other cell expressing PKC epsilon protein. Necropsy samples and pathology samples may also be used. Tissues comprising these cells may also be used. The cells may be fresh, cultured or frozen. Protein samples isolated from the cells or tissues may be used immediately in the diagnostic assay or methods for screening compounds or frozen for later use. In a preferred embodiment fibroblast cells are used. Fibroblast cells may be obtained by a skin punch biopsy.

Proteins may be isolated from the cells by conventional methods known to one of skill in the art. In a preferred method, cells isolated from a patient are washed and pelleted in phosphate buffered saline (PBS). Pellets are then washed with "homogenization buffer" comprising 50 nM NaF, ImM EDTA, 1 mM EGTA, 20 µg/ml leupeptin, 50 µg/ml pepstatin, 10 mM TRIS-HCl, pH = 7.4, and pelleted by centrifugation. The supernatant is discarded, and "homogenization buffer" is added to the pellet followed by sonication of the pellet. The protein extract may be used fresh or stored at -80°C for later analysis.

In the methods of the invention, the antibodies used in the disclosed immunoassays may be monoclonal or polyclonal in origin. The whole PKC epsilon protein or portions thereof used to generate the antibodies may be from natural or recombinant sources or generated by chemical synthesis. Natural Erk1/2 proteins can be isolated from biological samples by conventional methods. Examples of biological samples that may be used to isolate the PKC epsilon protein include, but are not limited to, skin cells, such as, fibroblasts, fibroblast cell lines, such as Alzheimer's disease fibroblast cell lines and control fibroblast cell lines which are commercially available through Coriell Cell Repositories, (Camden, N.J.) and listed in the National Institute of Aging 1991 Catalog of Cell Lines, National Institute of General Medical Sciences 1992/1993 Catalog of Cell Lines [(NIH Publication 92-201 1 (1992)].

The present invention is also directed to methods to screen and identify substances useful for the treatment or prevention of Alzheimer's disease. According to this embodiment, substances which reverse or improve the Alzheimer's disease-specific molecular biomarkers described herein (i.e. back to levels found in normal cells) would be identified and selected as substances which are potentially useful for the treatment or prevention of Alzheimer's disease.

By way of example, one such method of screening to identify therapeutic substances would involve the steps of contacting peripheral sample cells from an Alzheimer's disease patient with a substance being screened in the presence of any of the protein kinase C activators disclosed herein and then measuring any of the Alzheimer's disease-specific molecular biomarkers disclosed herein. An agent that reverses or improves the Alzheimer's disease-specific molecular biomarker back to levels found in normal peripheral cells (i.e. cells taken from a subject without Alzheimer's disease) would be identified and selected as a substance potentially useful for the treatment or prevention of Alzheimer's disease.

Compounds identified using the screening methods described herein may be formulated as pharmaceutical compositions for administration to subjects in need thereof.

A pharmaceutical composition or a compound (or a chemical derivative of a lead compound) identified using the screening methods disclosed herein can be administered safely by admixing with, for example, a pharmacologically acceptable carrier according to known methods to give a pharmaceutical composition, such as tablets (inclusive of sugar-coated tablets and film- coated tablets), powders, granules, capsules, (inclusive of soft capsules), liquids, injections, suppositories, sustained release agents and the like, for oral, subcutaneous, transdermal, transcutaneous or parenteral (e.g., topical, rectal or intravenous) administration.

Examples of pharmacologically acceptable carriers for use in the pharmaceutical compositionsinclude various conventional organic or inorganic carriers, including excipients, lubricants, binders and disintegrators for solid preparations, and solvents, solubilizers, suspending agents, isotonic agents, buffers, soothing agents, and the like for liquid preparations. Where necessary, conventional additives such as antiseptics, antioxidants, coloring agents, sweeteners, absorbents, moistening agents and the like can be used appropriately in suitable amounts.

A growing body of evidences suggests that a PKC signaling deficit is one of the major elements in causing the pathology of AD (Alkon et al., 2007, Liron et al., 2007, Choi et al., 2006). Previous findings have demonstrated that the distribution of PKC isozymes changes in the brains of AD patients (Shimohama et al., 1993, Masliah et al., 1990). PKC-α, PKC-γ and PKC-β were found lower in AD brains. Matsushima et al., 1996 have reported that in AD brain the PKC-ε level in both cytosolic and membranous fractions was found reduced, although PKC-δ and PKC-ξ levels were not changed, suggesting that among Ca2+-independent PKC isozymes, the alteration of PKC-ε is a specific event in AD brain and has a crucial role in AD pathophysiology. The major means of activating a-secretase mediated cleavage of APP is accomplished by PKC isozyme -α and -ε or indirectly through PKC mediated ERK 1/2 or both (Alkon et al., 2006; Skovronsky et al., 2000; Diaz-Rodrigez et al., 2002; Robinson and Cobb, 1997). The greatest risk factor for sporadic AD is age and it is associated with differential distribution of PKC isozymes in brain, impaired translocation, and reduced level of PKC anchoring protein RACK1 , (receptor of activated protein kinase C) (Battani et al., 1997).

**PKC-**ε **signaling deficit related to AD:** According to the amyloid hypothesis, AD is caused by the aggregation and accumulation of Aβ peptide forming amyloid plaque generated by the β- and γ-secretase pathway. Studies with human skin fibroblasts have documented anomalies in PKC isozyme function between AD patients and age matched controls (Van Huynh et al., 1989, Etcheberrugaray et al., 1993, Govoni et al., 1993, Favit et al., 1998). However, there has been previously no evidence showing decreased PKC-ε levels in peripheral tissues such as blood cells or skin fibroblasts of AD patients. To investigate, whether the lowered basal level of PKC-ε is AD-specific, AD patients were selected from both sporadic and familial AD cases with the presence of amyloid plaques and neurofibrilliary tangles in brain at autopsy and also compared with two different sets of non-AD dementia controls such as: (i) eight inherited Huntington's disease (HD) patients with strong evidence of non-AD characteristics with genetic identification of HD, and (ii) one Parkinson's disease and one fronto-temporal dementia patient fibroblasts. Therefore, the PKC-ε deficits were not associated with other non-AD dementia pathology. The basal levels of PKC-ε may also decrease with age. However, this study clearly demonstrated that the eleven AC (age-matched controls) had significantly higher PKC-ε levels. Therefore, the lowered basal level of PKC-ε in AD skin fibroblasts is due to the Alzheimer's pathology and not aging itself.

AD is a disease involving multiple pathological deficits and PKC is one of the major mechanistic controllers of cell survival, differentiation, and regulation. Among PKC's, PKC-ε controls synaptogenesis. PKC-ε is also reported to induce the transcription of low density receptor during cholesterol depletion (Mehta et al., 2002) and the LDL receptors have been suggested to play a role in transport and clearance of Aβ. In other studies, PKC ε activators were shown to enhance learning and memory as well as structurally specific synaptic changes in rat spatial maze learning (Hongpaisan and Alkon, 2007). Therefore, depletion in the total amount of PKC-ε in patients could lead to memory loss in AD. Furthermore, the transcript levels of the PKC- ε mRNA were also lower in the AD patient samples.

**Pathophysiological relevance of PKC-**ε **in AD:** It has been previously shown that PKC-α is degraded by Aβ treatment (Favit et al., 1998), and that Aβ alters the membrane translocation of PKC-a and PKC-ε in B 103 cells upon phorbol ester treatment (Lanni et al., 2004). It has also been shown that overexpression of PKC-ε reduced Aβ levels in transgenic mice (Choi et al., 2006; Hongpaisan et al., 201 1). Aβ oligomers which were > 100kDa molecular mass were found by to be highly toxic on primary rat neurons (Noguchi et al., 2009; Hoshi et al., 2003). Antibodies against these synthetic oligomers recognizes naive amylospheroids from AD patient brain (Noguchi et al., 2009), and hence these oligomers are pathologically significant with the disease. We have demonstrated (Fig. 4B) that these highly toxic oligomers (>100kDa) affected the PKC-ε levels in AC fibroblasts and converted it to AD phenotype. Treatment of the AC cells with these oligomers reduced the expression level of PKC-ε while it did not affect the AD cells. The AC-AD ratio of normalized PKC-ε in treated cells was found ∼1, while in untreated cells it was 1.4 (Fig. 4C).

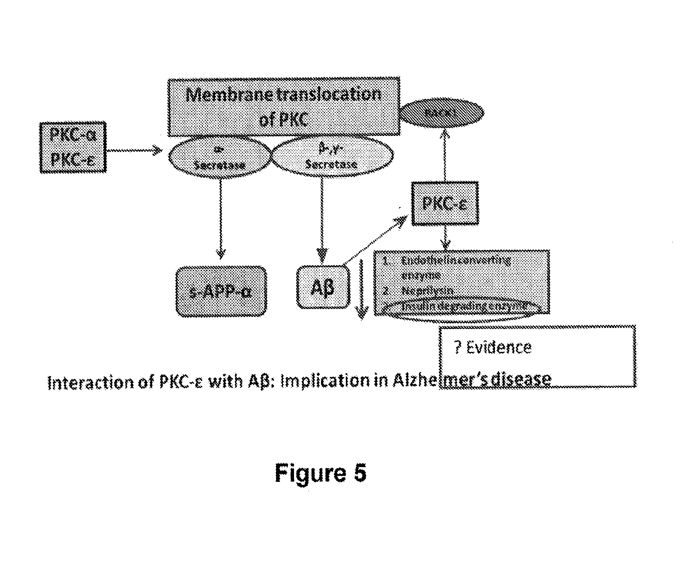

In AD pathology, over production of Aβ by higher β-,γ- sectretase activity and lower a-secretase activity might result in decreased amount of PKC-ε while on the other hand PKC-α and PKC-ε increase ot-secretase activity, as well as PKC-ε increases the activity of Aβ degrading enzymes. Since PKC-ε levels are found significantly lower in the AD fibroblasts compared to AC's and non- AD dementia's, therefore, AD related dysfunction of PKC-ε signaling and decreased basal amounts of PKC-ε in skin fibroblasts supports the possibility of peripheral PKC-ε as a biomarker for AD and PKC-ε activators as therapeutic candidates. It is possible that the different forms of toxic Aβ oligomers affect the PKC-ε levels in the cells, which is responsible for regulating the endothelin converting enzyme (ECE), that degrades Aβ. These proteins play an important role in Aβ clearance. Thus, a reasonable hypothesis is that abnormal accumulation of Aβ due to higher β-,γ- secretase activity causes a decrease in PKC-ε that then participates in a feedback loop (Fig. 5) to cause further Aβ elevation and synaptic loss.

**Deficiency of PKC**-ε **in AD fibroblasts and peripheral biomarker:** Though the gold standard for diagnosis of AD is postmortem analysis of neuropathological parameters, various laboratories are trying to find an effective diagnosis using peripheral tissue with the advantage of non-invasiveness, easy availability, low cost and most importantly early detection of the disease. The findings disclosed herein show that PKC signaling deficit is behind most of the AD elements. In aged animals, the PKC function is compromised with age specific distribution of PKC isozymes in brain, reduced translocation and reduced level of the RACK1 protein (Battani et al., 1997) and age is the most important risk factor in the case of sporadic AD. Over expression of PKC-ε has been shown to reduce the level of Aβ in AD transgenic mice (Choi et al., 2006). The inventors have surprisingly shown that PKC-ε is deficient in the peripheral cells of AD patients.

**Table: 1. Patient population: Description and identification of the human dermal fibroblasts**

| | **ID** | **Age** | **Sex** | **Description** |
|---|---|---|---|---|
| 1 | AG06844 | 59 YR | Male | Autopsy confirmed familial type 3 AD; 11 yrs of disease. |
| 2 | AG04159 | 52 YR | Female | Autopsy confirmed familial type 3 AD; 40 yrs of disease |
| 3 | AG06840 | 56 YR | Male | Autopsy confirmed familial type 3 AD; 1 yr of disease duration. |
| 4 | AG08245 | 75 YR | Male | Autopsy confirmed AD with no family history; 7yrs of disease |
| 5 | AG05770 | 70 YR | Male | Autopsy confirmed AD with no family history; 7 & 1/2 yrs disease |
| 6 | AG08527 | 61 YR | Male | Autopsy confirmed AD; 1 yr of disease |
| 7 | AG06263 | 67 YR | Female | Autopsy confirmed AD with no family history; 7yrs of disease |
| 8 | AG10788 | 87 YR | | Autopsy confirmed AD, familial; 17yrs of disease. Homozygous for Apoe4 |
| 9 | AG08259 | 90 YR | Male | Autopsy confirmed AD with no Family History, 3yrs of disease. |
| 10 | AG05810 | 79 YR | Female | The donor is clinically affected with severe late stage dementia, typical of AD. The APOE genotype of the donor subject is E3/E4 |
| 11 | A07714 | 56 YR | Female | Age matched control fibroblast |
| 12 | AG11734 | 50 YR | Female | Age matched control fibroblast |
| 13 | AG05840 | 55 YR | Female | Age matched control fibroblast |
| 14 | AG12927 | 66 YR | Female | Age matched control fibroblast |
| 15 | AG06242 | 71 YR | Male | Age matched control fibroblast |
| 16 | AG04461 | 66 YR | Male | Age matched control fibroblast |
| 17 | AG11363 | 74 YR | Female | Age matched control fibroblast |
| 18 | AG09977 | 63 YR | Female | Age matched control fibroblast |
| 19 | AG12998 | 65 YR | Male | Age matched control fibroblast |
| 20 | AG04560 | 59 YR | Male | Age matched control fibroblast |
| 21 | AG13358 | 72 YR | Female | Age matched control fibroblast |
| 22 | ND27760 | 55 YR | Female | Familial type 1 Parkinson's disease; Parkl. |
| 23 | GM2092 6 | 35 YR | Female | Inclusion body Myopathy with early-onset Paget disease and Frontotemporal dementia |
| 24 | GM0627 4 | 56 YR | Female | Huntington's disease |
| 25 | GM0217 3 | 52 YR | Female | Huntington's disease |
| 26 | GM0030 5 | 56 YR | Female | Huntington's disease; 10 yrs of disease duration. |
| 27 | GM0419 8 | 63 YR | Female | Huntington's disease inherited. |
| 28 | GM0503 1 | 60 YR | Male | Huntington's disease inherited. |
| 29 | GM0216 5 | 57 YR | Male | Huntington's disease inherited. 11 yrs of disease |
| 30 | GM0422 6 | 74YR | Male | Huntington's disease inherited. |
| 31 | GM0503 0 | 56YR | Male | Huntington's disease inherited. |

### Activators of PKC Epsilon

PKCε is the isozyme that most effectively suppresses Aβ production. Racci et al., Mol. Psychiatry. 2003; 8:209-216; and Zhu et al., Biochem. Biophys. Res. Commun. 2001 ; 285: 997-1006. Thus, isoform specific PKC activators are highly desirable as potential anti- Alzheimer's drugs. Specific activators are preferable to compounds such as bryostatin that show less specificity because non-specific activation of PKCδ or β could produce undesirable side effects.

Moreover, PKCε is also expressed at very low levels in all normal tissues except for brain. Mischak et al., J. Biol. Chem. 1993; 268: 6090-6096; Van Kolen et al., J. Neurochem. 2008;104: 1-13. The high abundance of PKCε in presynaptic nerve fibers suggest a role in neurite outgrowth or neurotransmitter release. Shirai et al., FEBS J. 2008; 275: 3988-3994). Therefore, effects of specific PKCε activators would be largely restricted to brain, and unlikely to produce unwanted peripheral side effects.

### PUFAs as PKC Activators

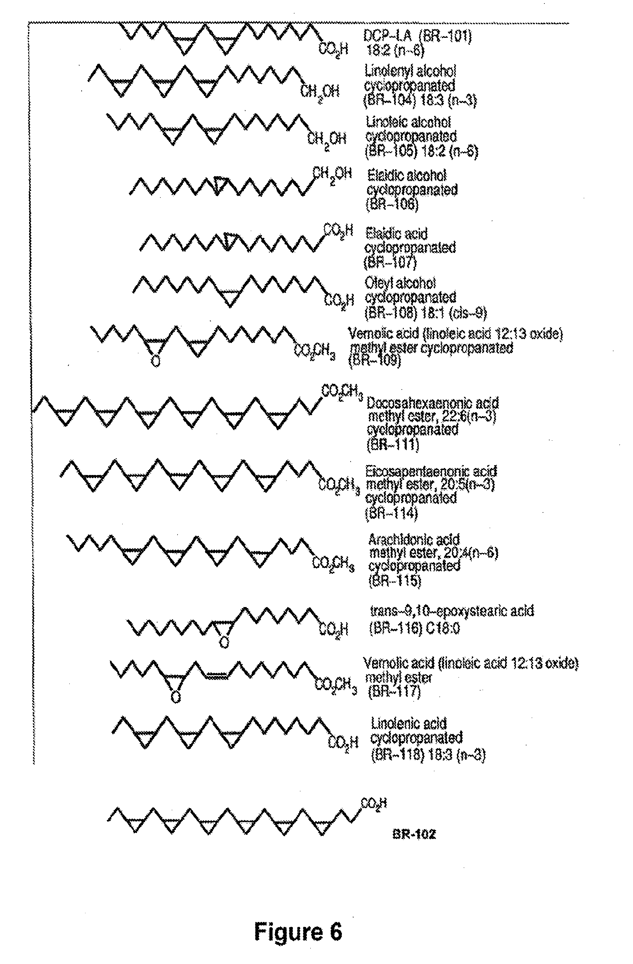

Some PUFAs, such as arachidonic acid (see Fig. 6), have been known for many years to be natural activators of PKC. Docosahexaenoic acid (DHA) is also a known activator of PKC and has recently been shown to slow the accumulation of Aβ and tau proteins associated with the brain-clogging plaques and tangles implicated in AD. Sahlin et al., Eur J Neurosci. 2007; 26(4):882-9.

Kanno et al. described effect of 8-[2-(2-pentyl- cyclopropylmethyl)-cyclopropyl]-octanoic acid (DCP-LA), a newly synthesized linoleic acid derivative with cyclopropane rings instead of *cis*-double bonds, on protein kinase C (PKC) activity. Journal of Lipid Research. 2007; 47: 1 146-1 156. DCP-LA activated PKCε, with a greater than 7-fold potency over other PKC isozymes. This indicates that DCP-LA is highly specific for PKCε. This compound also facilitated hippocampal synaptic transmission by enhancing activity of presynaptic acetylcholine receptors on the glutamatergic terminals or neurons. However, DCP-LA requires relatively high concentrations to produce its maximal effect.

WO 2002/50113 to Nishizaki et al., discloses carboxylic acid compounds and their corresponding salts having cyclopropane rings for LTP-like potentiation of synaptic transmission or for use as a cognition-enhancing drug or a drug to treat dementia. Their synthetic examples disclose preparation of esters but their experimental results teach the use of free acids. The reason is that the carboxylic acid group of the fatty acid starting material would react with the diethylzinc used in the Simmons-Smith reaction. The methyl ester acts as a protecting group and may be cleaved off by hydrolysis or allowed to remain as needed.

The caveats with the prior art finding include the necessity of administering high concentrations of to achieve the foregoing effects, non-specific activation of PKC isoforms, or rapid metabolism and sequestration of unmodified PUFAs into fat tissues and other organs where they are incorporated into triglycerides and chylomicrons. J Pharmacobiodyn. 1988;1 1(4):251-61. In addition use of unmodified PUFAs would have a myriad of adverse side effects. For example, arachidonic acid is a biochemical precursor to prostaglandins, thromboxanes, and leukotrienes, which have potent pro-inflammatory effects. This would be undesirable for treatment of Alzheimer's disease where the pathology likely involves inflammation. Other essential fatty acids may also possess a multitude of other biological effects, including enhancement of nitric oxide signaling, anti-inflammatory effects, and inhibition of HMG-CoA reductase, which would interfere with cholesterol biosynthesis.

Because of the limited existing options for treating both AD and stroke, new therapeutics that can selectively activate only the PKC isoforms that elicit neuroprotection are needed.

### PUFAs and MUFAs and Disease

A growing number of studies have suggested that omega-3 PUFAs can be beneficial for other mood disturbance disorders such as clinical depression, bipolar disorder, personality disorders, schizophrenia, and attention deficit disorders. Ross et al., Lipids Health Dis. 2007; 18;6:21. There is an abundance of evidence linking omega-3 fatty acids, particularly docosahexaenoic and eicosapentaenoic acids, and a healthy balance of omega-3 to omega-6 fatty acids, to lowering the risk of depression. Logan et al., Lipids Health Dis. 2004; 3: 25. Levels of omega-3 fatty acids were found to be measurably low and the ratio of omega-6 to omega-3 fatty acids were particularly high in a clinical study of patients hospitalized for depression. A recent study demonstrated that there was a selective deficit in docosahexaenoic in the orbitofrontal cortex of patients with major depressive disorder. McNamara et al. Biol Psychiatry. 2007;62(1):17-24. Several studies have also shown that subjects with bipolar disorder have lower levels omega-3 fatty acids. In several recent studies, omega-3 fatty acids were shown to be more effective than placebo for depression in both adults and children with bipolar depression. Osher and Belmaker, CNS Neurosci Ther. 2009;15(2): 128-33; Turnbull et al., Arch Psychiatr Nurs. 2008;22(5):305-1 1.

Extensive research also indicates that omega-3 fatty acids reduce inflammation and help prevent risk factors associated with chronic diseases such as heart disease, cancer, inflammatory bowel disease and rheumatoid arthritis. Calder et al., Biofactors. 2009;35(3):266-72; Psota et al., Am J Cardiol. 2006;98(4A):3i-18i; Wendel et al., Anticancer Agents Med Chem. 2009;9(4):457-70.

Monounsaturated fatty acids also have been shown to be beneficial in disorders. There is good scientific support for MUFA diets as an alternative to low-fat diets for medical nutrition therapy in Type 2 diabetes. Ros, American Journal of Clinical Nutrition. 2003; 78(3): 617S-625S. High-monounsaturated fatty acid diets lower both plasma cholesterol and triacylglycerol concentrations. Kris-Etherton et al., Am J Clin Nutr. 1999 Dec;70(6): 1009-15.

Cyclopropanated and epoxidized derivatives of PUFAs or MUFAs in which one, some, or all of the double bonds are replaced by a cyclopropane group or an epoxide group could be used. The terminal function may be a free carboxylic acid, or a methyl ester, ethyl ester, or some other alkyl ester with an aliphatic or aromatic alcohol. This alcohol specifically may also include glycerol and derivatives thereof. Glycerol derivatives are biologically important because the fatty acids are most frequently found conjugated to glycerol in the form of phosphatidylcholine, phosphatidylserine, or phosphatide acids. For example, triacylglycerols are compounds in which the carboxyl groups of fatty acids are esterified to the hydroxyls of all three carbons found in glycerol are referred to as triacylglycerols or triglycerides.

The purpose of esterifying the carboxylic acid is to facilitate transport across the blood-brain barrier by eliminating the negative charge. The purpose of an alcohol group is also to facilitate transport across the blood-brain barrier.

The fatty acid which forms the basis for the compounds is a polyunsaturated fatty acid having the following structure:

CH₃(CH₂)₄(CH=CHCH₂)x(CH₂)yCOOH

wherein X is between 2 and 6, and Y is between 2 and 6, and include methylene- or polymethylene-interrupted polyenes. Exemplary polyunsaturated fatty acids include linoleic acid, γ-linoleic, arachidonic acid, and adrenic acid having the following structures:

| | |
|---|---|
| Linoleic | CH₃(CH₂)₄(CH= CHCH₂)₂(CH₂)₆COOH |
| γ -Linolenic | CH₃(CH₂)₄(CH= CHCH₂)₃(CH₂)₃COOH |
| Arachidonic | CH₃(CH₂)₄(CH= CHCH₂)₄(CH₂)₂COOH |
| Adrenic | CH₃(CH₂)₄(CH= CHCH₂)₄(CH₂)₄COOH |

These are omega-6 PUFAs.

Furthermore, the fatty acid which forms the basis for the compounds is a polyunsaturated fatty acid having the following structure:

CH₃CH₂(CH= CHCH₂)x(CH₂)yCOOH

wherein X is between 2 and 6, and Y is between 2 and 6 and include methylene- or polymethylene-interrupted polyenes. Exemplary polyunsaturated fatty acids include a-lineoleic acid, docosahexaenoic acid, eicosapentaenoic acid, eicosatetraenoic acid having the following structures:

| | |
|---|---|
| Alpha-Linolenic | CH₃CH₂(CH= CHCH₂)₃(CH₂)₆COOH |
| Eicosatetraenoic | CH₃CH₂(CH=CHCH₂)₄(CH₂)₅COOH |
| Eicosapentaenoic | CH₃CH₂(CH=CHCH₂)₅(CH₂)₂COOH |
| Docosahexaenoic | CH₃CH₂(CH=CHCH₂)₆(CH₂)₂COOH |

These are known as omega-3 PUFAs.

In particular, the compound is an ester of a cw-PUFA, in which the hydroxyl group is replaced by an alkoxy group, and in which at least one of the double bonds has been cyclopropanated. The starting material for this embodiment has the following structures:

CH₃(CH₂)₄(CH=CHCH₂)x(CH₂)yCOOR or CH₃CH₂(CH= CHCH₂)x(CH₂)yCOOR

wherein R is the alkyl group from an alcohol including monohydric alcohols and polyhydric alcohols including but not limited to methanol, ethanol, propanol, butanol, pentanol, glycerol, mannitol, and sorbitol.

In a further embodiment, the compound contains at least three cyclopropanated double bonds.

In another embodiment, the fatty acid which forms the basis for the compounds is a monounsaturated fatty acid having the following structure:

CH₃(CH₂)xCH=CH(CH₂)yCOOH

wherein X and Y are odd numbers between 3 and 1 1.

Exemplary monounsaturated fatty acids that can be the basis for the compounds include *cis-* and trans- monounsaturated fatty acids such as oleic acid, elaidic acid, obtusilic acid, caproleic acid, lauroleic acid, linderic acid, myristoleic acid, palmitoleic acid, vaccenic acid, gadoleic acid, erucic acid, and petroselinic acid.

An ester , means a monoester or a polyester. Esters of fatty acids include methyl, propyl, and butyl esters, and also esters resulting from more complex alcohols such as propylene glycol. In non-limiting embodiments, R' is straight or branched and includes methyl, ethyl, propyl, isopropyl, butyl, isobutyl, secbutyl, tert-butyl, pentyl, hexyl, heptyl, octyl, nonyl, decyl, undecyl, dodecyl, tridecyl, and tetradecyl. An ester may also be formed from a fatty acid linked to a fatty alcohol in an ester linkage.

The ester can be a alcohol ester, including but not limited to an aliphatic alcohol ester. In one embodiment, the alcohol ester is a glycerol ester. Glycerol esters of fatty acids include glycerol fatty acid ester, glycerol acetic acid fatty acid ester, glycerol lactic acid fatty acid ester, glycerol citric acid fatty acid ester, glycerol succinic acid fatty acid ester, glycerol diacetyl tartaric acid fatty acid ester, glycerol acetic acid ester, polyglycerol fatty acid ester, and polyglycerol condensed ricinoleic acid ester.

In another specific embodiment, the compound is an alcohol of a cis-PUFA in which at least one of the double bonds has been cyclopropanated. In a further embodiment, the compound is an alcohol of a cis-PUFA which contains at least three cyclopropanated double bonds. These compounds include but are not limited to linoleic alcohol dicyclopropane (BR-105), or linolenic alcohol tricyclopropane (BR-104). In this embodiment, R' can be a normal or branched chain alcohol or a phenolic alcohol.

In another embodiment, the compound of the present invention, the compound is a *cis*-polyunsaturated fatty acid, or derivative thereof, in which at least one of the double bonds is replaced with an epoxyl group. In a further embodiment, the compound contains at least three epoxidized double bonds.

In a specific embodiment, the compound is an epoxidized ester of a *cis-*PUFA, including but not limited to a fatty alcohol ester. The esters can be the same esters as described above for the cyclopropanated PUFAS. In a further embodiment the alcohol can be an aliphatic alcohol ester, such as glycerol.

In another specific embodiment, the compound is an epoxidized *cis-*polyunsaturated fatty alcohol such as linoleic alcohol dicyclopropane or linolenic alcohol tricyclopropane. The alcohols can be the same as described above for the cyclopropanated PUFAS.

In another embodiment, the compound includes cyclopropanated or epoxidized lipids derived from cis-monounsaturated fatty acids (*cis*-monoenoic acids), such as oleic acid, elaidic acid, elaidic alcohol, oleyl alcohol, and 1 - monolinoleyl rac- glycerol. Exemplary compounds include eliadic alcohol cyclopropane (BR-106), eliadic acid cyclopropane (BR-107), and oleyl alcohol cyclopropane (BR-108).

A further embodiment includes cyclopropanated lipids derived from *cis-*monounsaturated fatty acids or unsaturated fatty acids, fatty acid esters, or fatty acid alcohols, containing one or more epoxide residues, such as vernolic acid methyl ester cyclopropane (*e.g*., BR-109).

In specific embodiments, the PUFAs which forms the basis of the cyclopropanated compounds used in the present invention include but are not limited to arachidonic acid (AA), docosahexaenoic acid (DHA), and eicosapentaenoic acid (EPA). Exemplary compounds for use in the method of the present invention include docahexaenonic acid methyl ester hexacyclopropane (BR-111); eicosapentaenoic acid methyl ester pentacyclopropane (BR-114); and arachidonic acid methyl ester tetracyclopropane (BR- 115).

In a further specific embodiment, the compound is a cyclopropanated PUFA derivative of docosahexaenoic acid having the following structure: 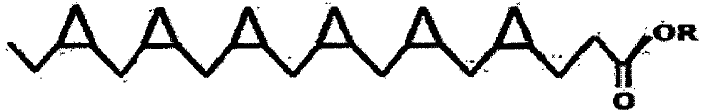 in which is h or an alkyl group. In a specific embodiment, R is CH3 (BR-III or DHA-CB6 methyl ester or methyl-3-(2-((2-((2-((2-((2-((2-ethylcyclopropyl)methyl)cyclopropyl)methyl)cyclopropyl)methyl)-cyclopropyl)methyl)cyclopropyl)methyl)cyclopropyl)propanoate.

In another specific embodiment, the PUFA derivative has the following structure:

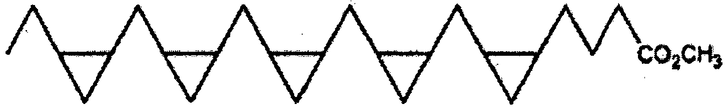

This compound is BR-114 (EPA-CP5 or methyl 4-(2((2-((2-((2-ethylcyclopropyl)methyl)cyclopropyl)methyl)cyclopropyl)methyl)cyclopropyl)methyl)-cyclopropyl)butanoate methyl ester).

In still another specific embodiment, the PUFA derivative has the following structure:

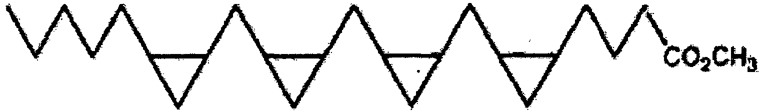

This compound is BR-115 (AA-CP4 or methyl 4-(2-((2-((2-((-pentylcyclopropyl)methyl)cyclopropyl)methyl)cyclopropyl)methyl)cyclopropyl)butanoat e methyl ester).

In yet another specific embodiment, the PUFA derivative has the following structure: in which R is H or an alkyl ester. In a specific embodiment, R is CH3.

Naturally cyclopropanated or epoxidized MUFAS or ester or alcohol derivatives thereof contemplated for use in the present invention include malvenic acid, vernolic acid, and sterculic acid. An exemplary compound is vernolic acid methyl ester (BR-117).

### Methods of synthesis

Fatty acids, and esters and alcohols thereof, can be obtained or made from purification from natural sources, e.g. , fish oil, flaxseed oil, soybeans, rapeseed oil, or algae, or synthesized using a combination of microbial enzymatic synthesis and chemical synthesis. As one example, fatty acid methyl esters can be produced by the transesterification of triglycerides of refined/edible type oils using methanol and an homogeneous alkaline catalyst.

Methods of cyclopropanation of double bonds in hydrocarbons are well known. As one example, the modified Simmons-Smith reaction is a standard method for converting double bonds to cyclopropanes. Tanaka and Nishizaki, Bioorg. Med. Chem. Let. 2003; 13: 1037-1040; Kawabata and Nishimura, J. Tetrahedron. 1967; 24: 53-58; and Denmark and Edwards, J. Org Chem. 1991 ; 56: 6974. In this reaction, treatment of alkenes with metal carbenoids, e.g. , methylene iodide and diethylzinc, result in cyclopropanation of the alkene. See also, Ito et al., Organic Syntheses. 1988; 6:327. Cyclopropanation of methyl esters of was also effected using diazomethane in the presence of palladium (II) acetate as catalyst. Gangadhar et al., Journal of the American Oil Chemists' Society. 1988; 65(4): 601-606.

Methods of epoxidation are also well known and typically involve reaction of fatty acids dioxiranes in organic solvents. Sonnet et al., Journal of the American Oil Chemists' Society. 1995; 72(2): 199-204. As one example, epoxidation of PUFA double bonds can be achieved using dimethyldioxirane (DMD) as the epoxidizing agent. Grabovskiy et al., Helvetica Chimica Acta. 2006; 89(10): 2243-53.

### EXAMPLES

### Example 1: Patient population and Cell culture

Human dermal fibroblasts from Alzheimer's disease patients (AD), non-AD dementia (Huntington's disease, Parkinson's disease and Frontotemporal dementia) patients, and age-matched control (AC) cases were obtained from the Coriell Institute of Medical Research (Camden, NJ). Fibroblast cells were maintained in DMEM with low glucose (Invitrogen, USA) supplemented with 10% FBS, and were grown to 100% confluence before experiments. Ten different examples of AD patients (four familial type and six sporadic; among these nine out of ten were autopsy confirmed), eleven AC and eight Huntington's disease, one Parkinson's disease and one Front temporal dementia were considered for the study (Table: 1). The average age of the AD cases was 69.6 ± 13.01 (SD) yrs, AC cases was 63.364 ± 7.65 (SD) yrs and non-AD dementia cases were 56.44 ± 9.7 (SD) yrs.

**Protein isolation:** Flasks containing cells were washed 3x with 1X PBS (pH 7.4) and the cells were collected using a cell scraper. The collected cells were transferred to 1.5 ml microcentrifuge tubes and centriiuged at 1000 rpm for 5 mins. The Cell pellet obtained was suspended in homogenizing buffer (10mM Tris pH 7.4, 1mM PMSF, 10mum EGTA, 10mM EDTA and 50mM NaF) and sonicated for 30 sees. The homogenate was centriiuged again at 4°C for 10mins at 10000 rpm and the supernatant was collected and transferred to a new tube for protein estimation. Total protein concentration was measured using a Bradford Protein assay Kit (Thermo Scientific, USA).

**Immunoblot analysis:** Protein lysates (20 µg of protein each) were boiled in 2X Laemmli buffer for 10 min and separated using a 4-20% gradient Tris- Glycine gels. Separated proteins were transferred to nitrocellulose membrane and the membrane was blocked in BSA at room temperature (RT) for 15 min and incubated with 1 : 2000 dilution anti-PKC-ε rabbit polyclonal antibody (Santa Cruz Biotechnology, Santa Cruz, CA.; Cat No: sc-214), and 1 : 5000 dilution anti-p-tubulin, class III rabbit monoclonal antibody (Millipore Corporation, Billerica MA, Cat No: 04-1049) for 1 hr at RT. After the incubation, the membrane fractions were washed 3x with standard western blot washing buffer and further incubated with alkaline phosphatase conjugated secondary antibody (Jackson Immunoresearch Laboratories, USA) at 1 : 10000 dilution for 45 min. The membrane fractions were finally washed 3x with standard western blot washing buffer and developed using the 1 -step NBT-BCIP substrate (Thermo Scientific, Rockford, IL). Signal intensities of the images were recorded in the ImageQuant RT- ECL (GE Life Sciences, Piscataway, NJ) and densitometric quantification was performed using the IMAL software (Blanchette Rockefeller Neurosciences Institute, Morgantown, WV). Intensities quantified in this way, for PKC-ε were normalized against β**-**tubulin for each lane.

**Immunofluorescence:** Fibroblasts cells were grown in chambered slides (Nunc, Rochester, NY) at low density. For immunofluorescence staining, the cells were washed 3 x with 1 ×PBS (pH 7.4) and fixed with 4% paraformaldehyde for 4 min. Following fixation, cells were blocked and permeabilized with 5% serum and 0.3% Triton X 100 in 1 x PBS for 30 min. Cells were washed 3x with 1 x PBS and incubated with rabbit polyclonal PKC-ε antibody (Santa Cruz Biotechnology, Santa Cruz, CA) for 1 hr at 1 : 100 dilution. After the incubation slides were washed 3 x in 1 x PBS and were incubated with the FITC anti-rabbit IgG (Jackson Immunoresearch Laboratories, USA) for 1 hr at 1 :400 dilution. Cells were washed and also stained with DAPI (4',6-diamidino-2'-phenylindole, dihydrochloride) (Thermo Scientific, USA). Finally, the slides were washed and mounted in glycerol PBS mounting solution and were viewed under the LSM 710 Meta confocal microscope (Zeiss, Germany) at 350 nra and 490 nm excitation and 470 nm and 525 nm emission for DAPI and FITC, respectively. Five individual fields were captured by 63 x oil lens magnification were analyzed for the mean fluorescence intensity (MFI) in each channel.

**RT-PCR:** RNA was isolated from ∼1 X 10⁶ cells using Trizol reagent (Invitrogen, USA) following manufacturer's protocol. Briefly, 2 µg of RNA was reverse transcribed using oligodT and Superscript III (Invitrogen, USA) at 50°C for 1 hr. Two 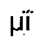 of the cDNA product was amplified using primers for PKC-ε (Forward Primer - AGCCTCGTTCACGGTTCTATGC, Reverse primer - GCAGTGACCTTCTGCATCCAGA), and β-tubulin (Forward Primer - TTGGGAGGTGATCAGCGATGAG, Reverse primer - CTCCAGATCCACGAGCACGGC) (Origene, Rockville, MD). The amplicons were analysed in an E-Gel (Invitrogen, USA) following 25 cycle amplification at 55°C annealing temperature. The gel image was documented using a Fuji Image gel scanner (FLA-9000, Fuji Film) and densitometric quantification was performed using the IMAL software (Blanchette Rockefeller Neurosciences Institute, Morgantown, WV). Data were represented as normalized ratio of PKC-ε OD (Optical Density) against β-tubulin OD for three independent experiments.

**Preparation of soluble oligomeric A**β: Oligomeric Aβ was prepared following the method described by Noguchi et al., (2009). Aβ generated by this method was reported to be highly neurotoxic, 10-15nm spherical Aβ assemblies termed as amylospheroids (ASPDs). For synthesis of ASPDs, Aβ₁₋₄₂ (Anaspec, USA) was dissolved in 1,1,1,3,3,3-hexafluro-2-propanol at 100µM concentration overnight at 4°C and then at 37°C for 3 hrs. Finally, the dissolved Aβ was lyophilized in aliquots (40nmol/tube). The lyophilized Aβ was dissolved in 50% PBS at 1 µM concentration and slowly rotated at 4°C for 14hrs. Flowing incubation the toxic ASPDs were purified using the 100kDa molecular mass cutoff filters (Ultrafree-MC, Millipore, USA). The retentates with molecular weight of > 100kda were used for treating fibroblasts.

**Treatment of skin fibroblasts with oligomeric Aβ₁₋₄₂:** Skin fibroblasts both from AD and AC cases were cultured for 7 days to 100% confluence. The confluent cells were treated with 500nM (final concentration) of ASPDs for 24 hrs at 37°C after they were 100% confluent. Following incubation, the cells were washed 3x with 1 xPBS (pH 7.4) and processed for western blotting as described earlier. The resulting band intensities for PKC-ε were quantified using ImageQuant RT-ECL (GE Life Sciences, USA) and densitometric analysis was performed using the IMAL software (Blanchette Rockefeller Neurosciences Institute, Morgantown, WV).

### Example 2: Synthesis of Fatty Acid Methyl Esters Cyclopropanated Fatty Acid Methyl Esters.

Synthesis of cyclopropanated fatty acids. Methyl esters of polyunsaturated fatty acids were cyclopropanated using the modified Simmons-Smith reaction using chloroiodomethane and diethylzinc (Tanaka et al., Bioorg. Med. Chem. Let. 2003; 13: 1037-40; Furukawa et al., Tetrahedron. 1967; 53-58; Denmark et al., J. Org. Chem. 1991 ; 56: 6974-81). All apparatus was baked at 60°C for 1 hr and dried using a flame with dry nitrogen. A 100 ml 3 -neck round bottom flask with a stirring bar and a temperature probe was surrounded by an ice-dry ice mixture and filled with 1.25g (4.24 mmol) linoleic acid methyl ester or docosahexaenoic acid methyl ester in 25 ml dichloromethane and bubbled with N₂. A 1M solution of diethylzinc (51 ml, 54.94 mmol) in hexane was added anaerobically using a 24-inch-long 20-gauge needle and the solution was cooled to -5°C. Diiodomethane (8.2 ml, 101.88 mmol) or chloroiodomethane (CICH2 I) was added dropwise, one drop per second, with constant stirring. The rate of addition was decreased if necessary to maintain the reaction mixture below 2°C. The reaction mixture became cloudy during the reaction and an insoluble white zinc product was liberated. The flask was sealed and the mixture was allowed to react for 1 hr and then allowed to come to room temperature gradually over 2 hr.

To prevent the formation of an explosive residue in the hood, diethylzinc was not evaporated off. The mixture was slowly poured into 100 ml of water under stirring to decompose any excess diethylzinc. Ethane was evolved. The mixture was centrifuged at 5000 rpm in glass centrifuge tubes and the upper aqueous layer discarded. The white precipitate was extracted with CH₂Cl₂ and combined with the organic phase. The organic phase was washed with water and centrifuged. The product was analyzed by silica gel G TLC using hexane plus 1% ethyl acetate and purified by chromatography on silica gel using increasing concentrations of 1-10% ethyl acetate in n- hexane and evaporated under nitrogen, leaving the methyl ester as a colorless oil.

The Simmons-Smith reaction preserves the stereochemistry of the starting materials. Furukawa et al., Tetrahedron. 1967; 53-58. Docosahexaenoic acid methyl ester was converted into DHA-CP6 in 90-95% yield. The product was a colorless oil with a single absorbance maximum at 202 nm in ethanol and no reaction with I₂. The IR spectrum showed cyclopropane ring absorption at 3070 and 1450 cm⁻¹. Under the same conditions, eicosapentaenoic acid methyl ester was converted to EPA-CP5, and arachidonic acid methyl ester was converted to AA-CP4. Linoleic acid methyl ester was converted to DCP-LA methyl ester which was identical to a known sample.

Hydrolysis of methyl ester. The methyl ester (0.15 g) was dissolved in 1 ml IN LiOH and 1 ml dioxane. Dioxane and methanol were added until it became homogeneous and the solution was stirred 60° overnight. The product was extracted in CH₂Cl₂ and centrifuged. The aqueous layer and white interface were re- extracted with water and washed until the white layer no longer formed. The product was evaporated under N₂ and purified by chromatography on silica gel. The product, a colorless oil, eluted in 20% EtOAc in n-hexane. Its purity was checked by TLC in 10% EtOAc/hexane and by C18 RP-HPLC using UV detection at 205 nm.

The epoxide groups can be introduced by conventional means, e.g., by oxidation of the appropriate alkene with m-chloroperbenzoic acid or t-butylhydroperoxide.

Other compounds synthesized include those depicted in Figure 1 (BR-101 through BR-118).

### Example 2: Activation of Purified PKC Epsilon using Docosahaexanoic Acid

Protein kinase C assay. Recombinant PKC (1 ng of alpha or epsilon isoform) was mixed with the BR-101 (DCP-LA) in the presence of 10 micromolar histones, 5 mM CaCl₂, 1.2 µg/µl phosphatidyl-L-serine, 0.18 µg/µl 1,2- dioctanoyl-sn-glycerol (DAG), 10 mM MgCl₂, 20 mM HEPES (pH 7.4), 0.8 mM EDTA, 4 mM EGTA, 4% glycerol, 8 µg/ml aprotinin, 8 µg/ml leupeptin, and 2 mM benzamidine. 0.5 micro Ci [^{γ32}P]ATP was added. The incubation mixture was incubated for 15 min at 37 degrees in a total volume of 10 microliters. The reaction was stopped by spotting the reaction mixtures on 1x2 cm strips of cellulose phosphate paper (Whatman P81) and immediately washing twice for 1 hr in 0.5% H₃PO₄. The cellulose phosphate strips were counted in a scintillation counter. In some experiments, phosphatidylserine, diacylglycerol, and/or calcium were removed.

DHA methyl ester was purchased from Cayman Chemical (Ann Arbor, ME). PKC isozymes were from Calbiochem (San Diego, CA). Purified PKCε was purchased from Calbiochem.

### Results

PKC measurements using purified PKCs showed that, at the lowest concentration tested (10 nM), compound BR-101 produced a 2.75-fold activation of PKCs. PKCα was not affected (data not shown). Compound BR-102 also selectively elicited activation of PKCε to about 1.75 fold over unactivated PKCε. The effectiveness of these compounds in activating PKCε at low concentrations suggests that they will be good therapeutic candidates.

### Example 3: Activation of Purified or Cellular PKC Epsilon using Other PKC Activators

Materials. Culture media were obtained from K-D Medical (Columbia, MD) or Invitrogen (Carlsbad, CA). Aβ1-42 was purchased from Anaspec (San Jose, CA). Polyunsaturated fatty acid methyl esters were obtained from Cayman Chemicals, Ann Arbor, MI. Other chemicals were obtained from Sigma-Aldrich Chemical Co. (St. Louis, MO). PKC isozymes were from Calbiochem (San Diego, CA). Purified PKCε was purchased from Calbiochem.

Cell culture. Rat hippocampal H19-7/IGF-IR cells (ATCC, Manassas, VA) were plated onto poly-L-lysine coated plates and grown at 35°C in DMEM/ 10% FCS for several days until about 50% coverage was obtained. The cells were then induced to differentiate into a neuronal phenotype by replacing the medium with 5 ml N₂ medium containing 10 ng/ml basic fibroblast growth factor at 39°C and grown in T-75 flasks at 37°C. Human SH-SY5Y neuroblastoma cells (ATCC) were cultured in 45% F12K / 45% MEM / 10% FCS. Mouse N2A neuroblastoma cells were cultured in DMEM/ 10% FCS without glutamine. Rat hippocampal neurons from 18-day-old embryonic

Sprague Dawley rat brains were plated on 12- or 96-well plates coated with poly-D-lysine (Sigma-Aldrich, St. Louis, MO) in B-27 neurobasal medium containing 0.5 mM glutamine and 25 µM glutamate (Invitrogen, Carlsbad, CA) and cultured for three days in the medium without glutamate. The neuronal cells were grown under 5% CO₂ in an incubator maintained at 37°C for 14 days.

All experiments on cultured cells were carried out in triplicate unless otherwise stated. All data points are displayed as mean ±SE. BR-101 (DCP-LA) was used as its free acid in all experiments, while BR-111 (DHA-CP6), BR-114 (EPA-CP5), and BR-116 (AA-CP4) were used as their methyl esters.

Protein kinase C assay. Rat hippocampal cells were cultured and scraped in 0.2 ml homogenization buffer (20 mM Tris-HCl, pH 7.4, 50 mM NaF, 1 µg/ml leupeptin, and 0.1 mM PMSF) and homogenized by sonication in a Marsonix microprobe sonicator (5 sec, 10W). To measure PKC, 10 µl of cell homogenate or purified PKC isozyme (purchased from Calbiochem) was incubated for 15 min at 37°C in the presence of 10µM histones, 4.89 mM CaCl₂ , 1.2µg/µl phosphatidyl-L-serine, 0.18µg/µl 1,2-dioctanoyl-sn-glycerol, 10 mM MgCl₂ , 20 mM HEPES (pH 7.4), 0.8 mM EDTA, 4 mM EGTA, 4% glycerol, 8 µg/ml aprotinin, 8 µg/ml leupeptin, and 2 mM benzamidine. 0.5µCi [^{γ-32}P]ATP was added and ³²P-phosphoprotein formation was measured by adsorption onto phosphocellulose as described previously. Nelson and Alkon, J. Neurochemistry. 1995; 65: 2350-57. For measurements of activation by BR-101 (DCP-LA) and similar compounds, PKC activity was measured in the absence of diacylglycerol and phosphatidylserine, as described by Kanno et al., and PKC δ, ε, η, and p were measured in the absence of added EGTA and CaCl2 , as described by Kanno et al., J. Lipid Res. 2006; 47: 1 146-50. Low concentrations of Ca²⁺ are used because high Ca²⁺ interacts with the PKC phosphatidylserine binding site and prevents activation. For measurements of bryostatin activation, 1,2-diacylglycerol was omitted unless otherwise stated.

### Results and Discussion

To determine their PKC isozyme specificity, the new compounds were preincubated with purified PKC for five minutes and the PKC activity was measured radiometrically. As shown for Example, 2, above, BR-101 (DCP-LA) was an effective activator of PKCs at 10 µM but had relatively small effects on the other PKC isoforms (data not shown). At higher concentrations BR-101 (DCP-LA) partially inhibited PKC5 (about 1 -100 µM) and activated PKCτ (50-100 µM) (data not shown).

BR-111 (DHA-CP6), BR-114 (EPA-CP5), and BR-115 (AA- CP4), which are cyclopropanated derivatives of docosahexaenoic acid, eicosapentaenoic acid, and arachidonic acid, respectively, activated purified PKCs to a similar extent. The concentration needed to activate PKC was approx. 100 times lower than for BR-101 (DCP-LA), suggesting higher affinity. Cyclopropanated linolenyl and linoleyl alcohols (BR-104 and BR-105), epoxystearic acid (BR-116), and vernolic acid methyl ester (BR- 117) had little or no effect on PKC. Cyclopropanated vernolic acid methyl ester (BR-109) inhibited PKCs at concentrations above 1 µM.

PKC activators that bind to the diacylglycerol binding site, including bryostatin, gnidimacrin, and phorbol esters, produce a transient activation of PKC activity, followed by a prolonged downregulation. Nelson et al., Trends in Biochem. Sci. 2009; 34: 136-45. This was confirmed in cultured rat hippocampal cells. Incubation of rat H19-7/IGF-IR cells with (0.04 nM and 0.2 nM) bryostatin produced a 2- fold activation that lasted 30 min, followed by a 20% downregulation that returned to baseline by 24h (data not shown). In contrast, PKC exposed to DCP-LA remained elevated for at least four hours. This sustained activation was only observed in primary neurons.

Even though bryostatin has a higher affinity for PKC than phorbol 12-myristate 13-acetate (PMA)(EC50 = 1.35 nM vs. 10 nM), bryostatin was much less effective than PMA at downregulating PKC. PKC activity is strongly downregulated by phorbol ester at 8h, while PKC in bryostatin-treated cells is at or near the baseline (data not shown). This difference may explain the increases in Aβ produced by PdBu reported by da Cruz e Silva et al. J. Neurochem. 2009: 108: 319-30. These investigators applied 1 µM PdBu to cultured COS cells for 8h and observed an increase in Aβ. This increase was attributed to downregulation of PKC by the phorbol ester, which is consistent with these results. Downregulation could not be measured for DCP-LA and related compounds.

### Example 4: Effects of PKC Activators on Aβ Production and Degradation

Cell culture. Cell culture was performed as described above for Example 3.

Aβ Measurement and Cell Viability Assay. Aβ was measured using an Aβ 1-42 human fluorimetric ELISA kit (Invitrogen) according to the manufacturer's instructions. Results were measured in a Biotek Synergy HT microplate reader. AlamarBlue and CyQuant NF (Invitrogen) according to the manufacturer's instructions.

### Results and Discussion

To measure the effects of PKCε activation on Aβ production, we used mouse neuro2a (N2a) neuroblastoma cells transfected with human APPS we/PS ID, which produce large quantities of Aβ. Petanceska et al., J Neurochem. 1996; 74: 1878- 84. Incubation of these cells for 24h with various concentrations of PKC activators, bryostatin, **BR-101** (DCP-LA) and **BR-111** (DHA-CP6) markedly reduced the levels of both intracellular and secreted Aβ. With bryostatin, which activates PKC by binding to the diacylglycerol-binding site, the inhibition was biphasic, with concentrations of 20 nM or higher producing no net effect. This may be explained by the ability of this class of PKC activators to downregulate PKC when used at high concentrations. In contrast, **BR- 101** (DCP-LA) and **BR-111** (DHA-CP6), which bind to PKC's phosphatidylserine site, showed monotonically increasing inhibition at concentrations up to 10 to 100 µM with no evidence of downregulation at higher concentrations.

To determine whether the reduced levels of Aβ caused by PKC activators were due to inhibition of Aβ synthesis or activation of Aβ degradation, we applied BR-111 (DHA-CP6) (0.01 to 10 µM) and low concentrations (100 nM) of exogenous monomeric Aβ-42 to cultured SH-SY5Y cells. This concentration of Aβ is too low to produce measurable toxicity or cell death. Since SH-SY5Y cells produce only trace amounts of Aβ, this experiment was an effective test of the ability of PKC activators to enhance Aβ degradation. By 24h, most of the Aβ had been taken up by the cells and the concentration of Aβ in the culture medium was undetectable. Addition of 0.01 to 10µM DHA-CP6 to the cells reduced the cellular levels of Aβ by 45-63%, indicating that the PKCE activator increased the rate of degradation of exogenous Aβ.

DHA-CP6, bryostatin, and DCP-LA had no effect on cell survival or on proliferation as measured by alamar Blue and CyQuant staining, indicating that the reduction in Aβ production did not result from cell proliferation or a change in cell survival.

### Example 5: Effects of PKC Activators on TACE Activity

TACE Assay. TACE was measured by incubating 5 µl cell homogenate, 3 µl buffer (50 mM Tris-HCl 7.4 plus 25 mM NaCl plus 4% glycerol), and 1 µl of 100 µM TACE substrate IV (Aβz-LAQAVRSSSR-DPa) (Calbiochem) for 20 min at 37° in 1.5-ml polypropylene centrifuge tubes(Jin et al., Anal. Biochem. 2002; 302: 269-75). The reaction was stopped by cooling to 4°C. The samples were diluted to 1 ml and the fluorescence was rapidly measured (ex = 320 nm, em = 420 nm) in a Spex Fluorolog 2 spectrofluorometer.

### Results and Discussion

Previous researchers reported that PKC activators such as phorbol 12-myristate 13-acetate produce large increases in TACE activity which correlated with increasd sAPPα and decreased Aβ, suggesting that TACE and BACE1 compete for availability of APP substrate, and that PKC activators shift the competition in favor of TACE. Buxbaum et al., J. Biol. Chem. 1998; 273: 27765-67; Etcheberrigaray et al., Proc. Natl. Acad. Sci. USA. 2006: 103:8215-20. However, many of these earlier studies were carried out in fibroblasts and other non-neuronal cell types, which appear to respond differently to PKC activators than neurons. For example, Etcheberrigaray et al. found that activation of PKC in human fibroblasts by 10 pM to 100 pM bryostatin increased the initial rate of α-secretase activity by 16-fold and 132-fold, respectively (Etcheberrigaray et al., Proc. Natl. Acad. Sci. USA. 2006). However, in human SH-SY5Y neuroblastoma cells, N2a mouse neuroblastoma cells, and primary neurons from rat hippocampus, PKC activators bryostatin, BR-101 (DCP-LA) and/or BR-111 (DHA-CP6) only produced small increases in TACE activity. This suggests that any reduction of Aβ levels in neurons by PKC activators must be caused by some other mechanism besides activation of TACE.

### Example 6: Effects of PKC Activators on Endothelin-Converting Enzyme Activity

**ECE assay.** SH-S757 neuroblastoma cells were incubated with bryostatin (0.27 nM), **BR-101** (DCP-LA) (1 µM), and **BR-111** (DHA-CP6) (1 µM). Endothelin-converting enzyme (ECE) was measured fluorimetrically using the method of Johnson and Ahn, Anal. Biochem. 2000; 286: 1 12-1 18. A sample of cell homogenate (20 µl) was incubated in 50 mM MES-KOH, pH 6.0, 0.01% C12E10 (polyoxyethylene- 10-lauryl ether), and 15 µM McaBK2 (7-Methoxycoumarin-4-acetyl [Ala7-(2,4- Dinitrophenyl)Lys9]-bradykinin trifluoroacetate salt) (Sigma-Aldrich). After 60 min at 37 °C, the reaction was quenched by adding trifluoroacetic acid to 0.5%. The sample was diluted to 1.4 ml with water and the fluorescence was measured at ex = 334 nm, em = 398 nm.

### Results and Discussion

Aβ can be degraded in vivo by a number of enzymes, including insulin degrading enzyme (insulysin), neprilysin, and ECE. Because PKCε overexpression has been reported to activate ECE (Choi et al., Proc. Natl. Acad. Sci. USA. 2006; 103: 8215-20), we examined the effect of PKC activators on ECE. Bryostatin, **BR-101** (DCP-LA), and **BR-111** (DHA-CP6) all produced a sustained increase in ECE activity. Since ECE does not possess a diacylglycerol-binding Cl domain, this suggests that the activation by bryostatin was not due to direct activation of ECE, but must have resulted from phosphorylation of ECE or some ECE-activating intermediate by PKC. This result also suggests that indirect activation ECE by PKC activators could be a useful means of reducing the levels of Aβ in patients.

An advantage of compounds such as the PUFA derivatives of the present invention which specifically activate PKCε is that they produce less downregulation than phorbol esters and similar 1 ,2-diacylglycerol (DAG) analogues. The biphasic response of PKC to DAG-based activators means that a PKC activator may reduce Aβ levels at one time point and increase them at another, da Cruz e Silva et al., J. Neurochem. 2009; 108: 319-330. Careful dosing and monitoring of patients would be required to avoid effects opposite to those that are intended. Because of the relative inability of this new class of PKC activators to downregulate PKC, this problem can be avoided.

### References

1. Alkon, D.L., Sun, M.K., Nelson, T. J., 2007. PKC signaling deficits: a mechanistic hypothesis for the origins of Alzheimer's disease. Trends Pharmacol. Sci. 28, 51- 60
2. Battaini, F., Pascale, A., Paoletti, R., Govoni, S., 1997. The role of anchoring protein RACK1 in PKC activation in the ageing rat brain. Trends Neurosci. 20, 410-5
3. Choi, D.S., Wang, D., Yu, G.Q., Zhu, G., Kharazia, V.N., Paredes, J.P., Chang, W.S., Deitchman, J.K., Mucke, L., Messing, R.O., 2006. PKC epsilon increases endothelin converting enzyme activity and reduces amyloid plaque pathology in transgenic mice. Proc. Natl. Acad. Sci. USA. 103, 8215-20
4. Citron, M., Vigo-Pelfrey, C, Teplow, D.B., Miller, C, Schenk, D., Johnston, J., Winblad, B., Venizelos, N., Lannfelt, L., Selkoe, D.J., 1994. Excessive production of amyloid beta-protein by peripheral cells of symptomatic and presymptomatic patients carrying the Swedish familial Alzheimer disease mutation. Proc. Natl. Acad. Sci. USA. 91, 1 1993-7
5. Cole, G., Dobkins, K.R., Hansen, L.A., Terry, R.D., Saitoh, T., 1988. Decreased levels of protein kinase C in Alzheimer brain. Brain Res. 452, 165-74
6. Dehvari, N., Isacsson, O., Winblad, B., Cedazo-Minguez, A., Cowburn, R.F., 2008. Presenilin regulates extracellular regulated kinase (Erk) activity by a protein kinase C alpha dependent mechanism. Neurosci. Lett. 436,77-80.
7. Diaz-Rodriguez, E., Esparis-Ogando, A., Montero, J.C., Yuste, L., Pandiella, A., 2000. Stimulation of cleavage of membrane proteins by calmodulin inhibitors. Biochem. J. 346, 359-67.
8. Etcheberrigaray, R., Ito, E., Kim, C.S., Alkon, D.L., 1994. Soluble beta-amyloid induction of Alzheimer's phenotype for human fibroblast K+ channels. Science 264, 276-9
9. Etcheberrigaray, R., Ito, E., Oka, K., Tofel-Grehl, B., Gibson, G.E., Alkon, D.L., 1993. Potassium channel dysfunction in fibroblasts identifies patients with Alzheimer disease. Proc. Natl. Acad. Sci. USA. 90, 8209-13
10. Favit, A., Grimaldi, M., Nelson, T.J., Alkon, D.L., 1998. Alzheimer's-specific effects of soluble beta-amyloid on protein kinase C-alpha and -gamma degradation in human fibroblasts. Proc. Natl.Acad. Sci. USA. 95, 5562-7
11. Govoni, S., Bergamaschi, S., Racchi, M., Battaini, F., Binetti, G., Bianchetti, A., Trabucchi, M., 1993. Cytosol protein kinase C downregulation in fibroblasts from Alzheimer's disease patients. Neurology 43, 2581-6
12. Guerreiro, R.J., Santana, I., Bras, J.M., Santiago, B., Paiva, A., Oliveira, C, 2007. Peripheral inflammatory cytokines as biomarkers in Alzheimer's disease and mild cognitive impairment. Neurodegener. Dis. 4, 406-12
13. Hongpaisan, J., Alkon, D.L., 2007. Astructural basis for enhancement of longterm associative memory in single dendritic spines regulated by PKC. Proc. Natl. Acad. Sci. USA. 104, 19571-19576
14. Hongpaisan, J., Sun, M.K., Alkon, D.L., 201 1. PKC-ε activation prevents synaptic loss, Aβ elevation, and cognitive deficits in Alzheimer's disease transgenic mice. J. Neurosci. 31,630-643..
15. Hoshi, M, Sato, M., Matsumoto, S., Noguchi, A., Yasutake, K., Yoshida, N., Sato, K., 2003. Spherical aggregates of beta-amyloid (amylospheroid) show high neurotoxicity and activate tau protein kinase I/glycogen synthase kinase-3beta. Proc. Natl. Acad. Sci. USA. 100, 6370-5
16. Hoshino, M., Dohmae, N., Takio, K., Kanazawa, I., Nukina, N., 2003. Identification of a novel amino-terminal fragment of amyloid precursor protein in mouse neuroblastoma Neuro2a cell. Neurosci. Lett 353, 135-8
17. Ito, E., Oka, K., Etcheberrigaray, R., Nelson, T.J., McPhie, D.L., Tofel-Grehl, B., Gibson, G.E., Alkon, D.L., 1994. Internal Ca2+ mobilization is altered in fibroblasts from patients with Alzheimer disease. Proc. Natl. Acad. Sci.USA. 91 , 534-8
18. Joachim, C.L., Mori, H., Selkoe, D.J., 1989. Amyloid beta-protein deposition in tissues other than brain in Alzheimer's disease. Nature 341 , 226-30
19. Johnston. J. A., Cowburn, R.F., Norgren, S., Wiehager, B., Venizelos, N., Winblad, B., Vigo-Pelfrey, C, Schenk, D., Lannfelt, L., O'Neill, C, 1994. Increased beta-amyloid release and levels of amyloid precursor protein (APP) in fibroblast cell lines from family members with the Swedish Alzheimer's disease APP670/671 mutation. FEBS. Lett. 354, 274-8
20. Jolly-Tornetta, C, Wolf, B.A., 2000. Protein kinase C regulation of intracellular and cell surface amyloid precursor protein (APP) cleavage in CH0695- cells. Biochemistry 39, 15282-90
21. Khan, T.K., Alkon, D.L., 2006. An internally controlled peripheral biomarker for Alzheimer's disease: Erk1 and Erk2 responses to the inflammatory signal bradykinin. Proc. Natl. Acad. Sci. USA. 103, 13203-7
22. Khan, T.K., Nelson, T.J., Verma, V.A., Wender, P.A., Alkon, D.L., 2009. A cellular model of Alzheimer's disease therapeutic efficacy: PKC activation reverses Abeta-induced biomarker abnormality on cultured fibroblasts. Neurobiol. Dis. 34, 332-9
23. Khan, T.K., Alkon, D.L., 2010. Early diagnostic accuracy and pathophysiologic relevance of an autopsy-confirmed Alzheimer's disease peripheral biomarker. Neurobiol. Aging. 31 (6),889-900.
24. Kinouchi, T., Sorimachi, H., Maruyama, K., Mizuno, K., Ohno, S., Ishiura, S., Suzuki, K., 1995. Conventional protein kinase C (PKC)-alpha and novel PKC epsilon, but not -delta, increase the secretion of an N-terminal fragment of Alzheimer's disease amyloid precursor protein from PKC cDNA transfected 3Y1 fibroblasts. FEBS. Lett. 364, 203-6
25. Lanni, C, Mazzucchelli, M, Porrello, E., Govoni, S., Racchi, M., 2004. Differential involvement of protein kinase C alpha and epsilon in the regulated secretion of soluble amyloid precursor protein. Eur. J. Biochem. 271, 3068-75
26. Liron, T., Chen, L.E., Khaner, H., Vallentin, A., Mochly-Rosen, D., 2007. Rational design of a selective antagonist of epsilon protein kinase C derived from the selective allosteric agonist, pseudo-RACK peptide. J. Mol. Cell Cardiol. 42, 835-41
27. Liron, T., Seraya, C.B., Ish-Shalom, M., Souroujon, M.C., Neumann, D., 2007. Overexpression of amyloid precursor protein reduces epsilon protein kinase C levels. Neuroscience 146, 152-9
28. Masliah, E., Cole, G.M., Shimohama, S., Hansen, L.A., DeTeresa, R., Terry, R.D., Saitoh, T., 1990. Differential involvement of protein kinase C isozymes in Alzheimer's disease. J. Neurosci. 10, 21 13-24
29. Masliah, E., Cole, G., Hansen, L.A., Mallory, M., Albright, T., Terry, R.D., Saitoh, T., 1991 . Protein kinase C alteration is an early biochemical marker in Alzheimer's disease. J. Neurosc. 1 1 , 2759-2767
30. Matsushima, H., Shimohama, S., Chachin, M., Taniguchi, T., Kimura, J., 1996. Ca2+-dependent and Ca2+-independent protein kinase C changes in the brain of patients with Alzheimer's disease. J. Neurochem. 67, 317-23
31. Mehta, K.D., Radominska-Pandya, A., Kapoor, G.S., Dave, B., Atkins, B.A., 2002. Critical role of diacylglycerol- and phospholipid-regulated protein kinase C epsilon in induction of low-density lipoprotein receptor transcription in response to depletion of cholesterol. Mol. Cell. Biol. 22, 3783-93
32. Noguchi, A., Matsumura, S., Dezawa, M., Tada, M., Yanazawa, M, Ito, A" Akioka, M, Kikuchi, S., Sato, M., Ideno, S., Noda, M., Tukunari, A., Muramatsu, S., Itokazu, Y., Sato, K., Takahashi, H., Teplow, D.B., Nabeshima, Y., Kakita, A., Imahori, K., Hoshi, M., 2009. Isolation and characterization of patient-derived, toxic, high mass amyloid beta-protein (Abeta) assembly from Alzheimer disease brains. J. Biol. Chem. 284, 32895-905.
33. Ray, S., Britschgi, M., Herbert, C, Takeda-Uchimura, Y., Boxer, A., Blennow, K., Friedman, L.F., Galasko, D.R., Jutel, M, Karydas, A., Kaye, J.A., Leszek, J., Miller, B.L., Minthon, L., Quinn, J.F., Rabinovici, G.D., Robinson, W.H., Sabbagh, M.N., So, Y.T., Sparks, D.L., Tabaton, M., Tinklenberg, J., Yesavage, J.A., Tibshirani, R., Wyss-Coray, T., 2007. Classification and prediction of clinical Alzheimer's diagnosis based on plasma signaling proteins. Nat. Med. 13, 1359-62
34. Robinson, M.J., Cobb, M.H., 1997. Mitogen-activated protein kinase pathways. Curr. Opin. Cell. Biol. 9, 180-6
35. Shimohama, S., Narita, M., Matsushima, H., Kimura, J., Kameyama, M., Hagiwara, M., Hidaka, H., Taniguchi, T., 1993. Assessment of protein kinase C isozymes by two-site enzyme immunoassay in human brains and changes in Alzheimer's disease. Neurology 43, 1407-13
36. Skovronsky, D.M., Moore, D.B., Milla, M.E., Doms, R.W., Lee, V.M., 2000. Protein kinase C-dependent alpha-secretase competes with beta-secretase for cleavage of amyloid-beta precursor protein in the trans-golgi network. J. Biol. Chem. 275, 2568-75
37. Slack, B.E., Nitsch, R.M., Livneh, E., Kunz, G.M., Jr., Breu, J., Eldar, H., Wurtman, R.J., 1993. Regulation by phorbol esters of amyloid precursor protein release from Swiss 3T3 fibroblasts overexpressing protein kinase C alpha. J. Biol. Chem. 268, 21097-101
38. Slack, B.E., Nitsch, R.M., Livneh, E., Kunz, G.M., Jr., Eldar, H., Wurtman, R.J., 1993. Regulation of amyloid precursor protein release by protein kinase C in Swiss 3T3 fibroblasts. Ann. N. Y. Acad. Sci. 695, 128-31
39. Soininen, H., Helkala, EX., Laulumaa, V., Soikkeli, R., Hartikainen, P., Riekkinen, P.J., 1992. Cognitive profile of Alzheimer patients with extrapyramidal signs: a longitudinal study. J. Neural Transm. Park .Dis. Dement .Sect. 4, 241-54
40. Soininen, H., Laulumaa, V., Helkala, E.L., Hartikainen, P., Riekkinen, P.J., 1992. Extrapyramidal signs in Alzheimer's disease: a 3-year follow-up study. J. Neural. Transm. Park .Dis Dement. Sect. 4, 107-19
41. Soininen, H., Reinikainen, K., Partanen, J., Mervaala, E., Paljarvi, L., Helkala, E.L., Riekkinen, P., Sr., 1992. Slowing of the dominant occipital rhythm in electroencephalogram is associated with low concentration of noradrenaline in the thalamus in patients with Alzheimer's disease. Neurosci. Lett. 137, 5-8
42. Van Huynh, T., Cole, G., Katzman, R., Huang, K.P., Saitoh, T., 1989. Reduced protein kinase C immunoreactivity and altered protein phosphorylation in Alzheimer's disease fibroblasts. Arch. Neurol 46, 1 195-9
43. Yeon, S.W., Jung, M.W., Ha, M.J., Kim, S.U., Huh, K., Savage, M.J., Masliah, E., Mook-Jung, I., 2001. Blockade of PKC epsilon activation attenuates phorbol ester-induced increase of alpha-secretase-derived secreted form of amyloid precursor protein. Biochem. Biophys. Res. Commun. 280, 782-7
44. Zhao, W.Q., Feng, C, Alkon, D.L., 2003. Impairment of phosphatase 2A contributes to the prolonged MAP kinase phosphorylation in Alzheimer's disease fibroblasts. Neurobiol. Dis. 14, 458-69
45. Zhao, W.Q., Ravindranath, L., Mohamed, A.S., Zohar, O., Chen, G.H., Lyketsos, C.G., Etcheberrigaray, R., Alkon, D.L., 2002. MAP kinase signaling cascade dysfunction specific to Alzheimer's disease in fibroblasts. Neurobiol. Dis. 11 , 166-83
46. Zhu, G., Wang, D., Lin, Y.H., McMahon, T., Koo, E.H., Messing, R.O., 2001. Protein kinase C epsilon suppresses Abeta production and promotes activation of alpha-secretase. Biochem. Biophys. Res. Coramun. 285, 997-1006

## Claims

1. A in vitro method of diagnosing Alzheimer's Disease, said method comprising the steps of:
a) determining the PKC epsilon level in one or more peripheral cells of a human subject; and
b) comparing the PKC epsilon level in one or more peripheral cells of said human subject to the PKC epsilon level in one or more peripheral cells of a control subject;
wherein said method is indicative of Alzheimer's Disease if the PKC epsilon level in one or more peripheral cells of said human subject is lower than the PKC epsilon level in one or more peripheral cells of said control subject.

2. The method of claim 1, wherein said PKC epsilon level is a PKC epsilon protein level or a PKC epsilon activity level.

3. The method of claim 1, wherein said control subject does not have Alzheimer's Disease.

4. The method of claim 1, wherein the absence of Alzheimer's Disease is indicated if said PKC epsilon level in one or more peripheral cells of said human is greater than or equal to the PKC epsilon level in one or more peripheral cells of said control subject.

5. A in vitro method of diagnosing Alzheimer's Disease comprising the steps of:
b) determining the PKC epsilon level in one or more peripheral cells of a human subject;
c) contacting said one or more peripheral cells of step (a) with an agent that is a PKC epsilon activator;
d) determining the PKC epsilon level in said one or more peripheral cells in step (c) after said contacting in step (c); wherein Alzheimer's Disease is indicated if the PKC epsilon level determined in step (d) is greater that the PKC epsilon level determined in step (b).

6. The method of claim 5, wherein the absence of Alzheimer's Disease is indicated if said PKC epsilon level in determined in step (d) is equal to or less than the PKC epsilon level determined in step (b).

7. A in vitro method of determining or monitoring Alzheimer's Disease progression comprising the steps of:
a) determining the PKC epsilon level in one or more peripheral cells of a human subject;
b) comparing the PKC epsilon level in one or more peripheral cells of said human subject to the PKC epsilon level in one or more peripheral cells of a control subject; and
c) determining or monitoring said Alzheimer's Disease progression based on said comparison in step (b).

8. The method of claim 7, wherein said PKC epsilon level is a PKC epsilon protein level or a PKC epsilon activity level.

9. The method of claim 7, wherein the PKC epsilon level increases in one or more peripheral cells_of said human subject as Alzheimer's Disease progression is reversed.

10. A in vitro method for elevating the PKC epsilon protein level in a cell, comprising the step of contacting one or more human peripheral cells with an amount of a PKC activator effective to elevate the PKC epsilon protein level in said peripheral cell compared to an uncontacted human peripheral cell.

11. A in vitro method of diagnosing Alzheimer's Disease comprising the steps of:
b) determining the PKC epsilon level in one or more peripheral cells of a human subject;
c) contacting said one or more peripheral cells of step (a) with an Aβ peptide;
d) determining the PKC epsilon level in said one or more peripheral cells in step (c) after said contacting in step (c);
wherein Alzheimer's Disease is indicated if the PKC epsilon level determined in step (d) is not significantly different from the PKC epsilon level determined in step (b).

12. The method of claim 11, wherein the absence of Alzheimer's Disease is indicated if said PKC epsilon level in determined in step (d) is less than the PKC epsilon level determined in step (b).

13. A in vitro method of identifying a compound useful for the treatment of Alzheimer' Disease comprising:
b) determining the PKC epsilon level in one or more peripheral cells of an Alzheimer's Disease subject;
c) contacting said peripheral cells with a candidate compound;
d) determining the PKC epsilon level in said one or more peripheral cells after said contacting step (c);
wherein said candidate compound is identified as a compound useful for the treatment of Alzheimer's Disease if the PKC epsilon level determined in step (d) is greater than the PKC epsilon level determined in step (b).

## Patentansprüche

1. In-vitro-Verfahren zum Diagnostizieren der Alzheimer-Krankheit, wobei das Verfahren die Schritte umfasst:
a) Bestimmen des PKC-epsilon-Levels in ein oder mehreren peripheren Zellen von einem humanem Subjekt; und
b) Vergleichen des PKC-epsilon-Levels in ein oder mehreren peripheren Zellen des humanen Subjekts mit dem PKC-epsilon-Level in ein oder mehreren peripheren Zellen von einem Kontrollsubjekt;
wobei das Verfahren für eine Alzheimer-Krankheit anzeigend ist, wenn der PKC-epsilon-Level in ein oder mehreren peripheren Zellen des humanen Subjekts geringer ist als der PKC-epsilon-Level in ein oder mehreren peripheren Zellen des Kontrollsubjekts.

2. Verfahren nach Anspruch 1, wobei der PKC-epsilon-Level ein PKC-epsilon-Proteinlevel oder ein PKC-epsilon-Aktivitätslevel ist.

3. Verfahren nach Anspruch 1, wobei das Kontrollsubjekt keine Alzheimer-Krankheit hat.

4. Verfahren nach Anspruch 1, wobei das Fehlen der Alzheimer-Krankheit angezeigt ist, wenn der PKC-epsilon-Level in ein oder mehreren peripheren Zellen des humanen Subjekts höher oder gleich ist als der PKC-epsilon-Level in ein oder mehreren peripheren Zellen des Kontrollsubjekts.

5. In-vitro-Verfahren zum Diagnostizieren der Alzheimer-Krankheit, umfassend die Schritte:
b) Bestimmen des PKC-epsilon-Levels in ein oder mehreren peripheren Zellen von einem humanem Subjekt; und
c) In-Kontakt-Bringen der ein oder mehreren peripheren Zellen aus dem Schritt (a) mit einem Mittel, das ein PKC-epsilon-Aktivator ist;
d) Bestimmen des PKC-epsilon-Levels in den ein oder mehreren peripheren Zellen des Schritts (c) nach dem In-Kontakt-Bringen in dem Schritt (c); wobei eine Alzheimer-Krankheit anzeigt ist, wenn der PKC-epsilon-Level, der im Schritt (d) bestimmt wird, größer ist als der PKC-epsilon-Level, der im Schritt (b) wird.

6. Verfahren nach Anspruch 5, wobei das Fehlen der Alzheimer-Krankheit angezeigt ist, wenn der PKC-epsilon-Level, der im Schritt (d) bestimmt wird, gleich oder geringer ist als der PKC-epsilon-Level, der im Schritt (b) bestimmt wird.

7. In-vitro-Verfahren zum Bestimmen oder Überwachung des Fortschritts der Alzheimer-Krankheit, umfassend die Schritte:
a) Bestimmen des PKC-epsilon-Levels in ein oder mehreren peripheren Zellen von einem humanem Subjekt;
b) Vergleichen des PKC-epsilon-Levels in ein oder mehreren peripheren Zellen des humanen Subjekts mit dem PKC-epsilon-Level in ein oder mehrere peripheren Zellen von einem Kontrollsubjekt; und
c) Bestimmen oder Überwachen des Fortschritts der Alzheimer-Krankheit basierend auf dem Vergleich im Schritt (b).

8. Verfahren nach Anspruch 7, wobei der PKC-epsilon-Level ein PKC-epsilon-Proteinlevel oder ein PKC-epsilon-Aktivitätslevel ist.

9. Verfahren nach Anspruch 7, wobei der PKC-epsilon-Level in ein oder mehreren peripheren Zellen des humanen Subjekts ansteigt während der Fortschritt der Alzheimer-Krankheit rückgängig gemacht wird.

10. In-vitro-Verfahren zum Anheben des PKC-epsilon-Proteinlevels in einer Zelle, umfassend den Schritt des In-Kontakt-Bringens von ein oder mehreren humanen peripheren Zellen mit einer Menge an einem PKC-Aktivator, die wirksam ist, um den PKC-epsilon-Proteinlevel in der peripheren Zelle verglichen mit einer nicht in Kontakt gebrachten humanen peripheren Zelle anzuheben.

11. In-vitro-Verfahren zum Diagnostizieren der Alzheimer-Krankheit, umfassend die Schritte:
b) Bestimmen des PKC-epsilon-Levels in ein oder mehreren peripheren Zellen von einem humanem Subjekt;
c) In-Kontakt-Bringen der ein oder mehreren Zellen aus dem Schritt (a) mit einem Aß-Peptid;
d) Bestimmen des PKC-epsilon-Levels in den ein oder mehreren peripheren Zellen des Schritts (c) nach dem In-Kontakt-Bringen in dem Schritt (c);
wobei eine Alzheimer-Krankheit anzeigt ist, wenn der PKC-epsilon-Level, der im Schritt (d) bestimmt wird, nicht signifikant von dem PKC-epsilon-Level, der im Schritt (b) bestimmt wird, verschieden ist.

12. Verfahren nach Anspruch 11, wobei das Fehlen einer Alzheimer-Krankheit anzeigt ist, wenn der PKC-epsilon-Level, der im Schritt (d) bestimmt wird, geringer ist als der PKC-epsilon-Level, der im Schritt (b) bestimmt wird.

13. In-vitro-Verfahren zum Identifizieren einer Verbindung, die zur Behandlung der Alzheimer-Krankheit nützlich ist, umfassend das:
b) Bestimmen des PKC-epsilon-Levels in ein oder mehreren peripheren Zellen von einem Subjekt mit Alzheimer-Krankheit;
c) In-Kontakt-Bringen der peripheren Zellen mit einer Kandidatenverbindung;
d) Bestimmen des PKC-epsilon-Levels in den ein oder mehreren peripheren Zellen nach dem Schritt (c) des In-Kontakt-Bringens;
wobei die Kandidatenverbindung als eine Verbindung identifiziert wird, die für die Behandlung der Alzheimer-Krankheit nützlich ist, wenn der PKC-epsilon-Level, der im Schritt (d) bestimmt wird, größer ist als der PKC-epsilon-Level, der im Schritt (b) bestimmt wird.

## Revendications

1. Procédé *in vitro* pour établir le diagnostic de la maladie d'Alzheimer, ledit procédé comprenant les étapes qui consistent à :
a) déterminer le taux de la PKC epsilon dans une ou plusieurs cellules périphériques d'un sujet humain ; et
b) comparer le taux de la PKC epsilon dans une ou plusieurs cellules périphériques dudit sujet humain au taux de la PKC epsilon dans une ou plusieurs cellules périphériques d'un sujet de contrôle ;
dans lequel ledit procédé indique la maladie d'Alzheimer si le taux de la PKC epsilon dans une ou plusieurs cellules périphériques dudit sujet humain est inférieur au taux de la PKC epsilon dans une ou plusieurs cellules périphériques dudit sujet de contrôle.

2. Procédé selon la revendication 1, dans lequel ledit taux de la PKC epsilon est un taux protéique de la PKC epsilon ou un taux d'activité de la PKC epsilon.

3. Procédé selon la revendication 1, dans lequel ledit sujet de contrôle n'est pas atteint de la maladie d'Alzheimer.

4. Procédé selon la revendication 1, dans lequel l'absence de la maladie d'Alzheimer est indiquée si ledit taux de la PKC epsilon dans une ou plusieurs cellules périphériques dudit humain est supérieur ou égal au taux de la PKC epsilon dans une ou plusieurs cellules périphériques dudit sujet de contrôle.

5. Procédé *in vitro* pour établir le diagnostic de la maladie d'Alzheimer, comprenant les étapes qui consistent à :
b) déterminer le taux de la PKC epsilon dans une ou plusieurs cellules périphériques d'un sujet humain ;
c) mettre en contact lesdites une ou plusieurs cellules périphériques de l'étape (a) avec un agent qui est un activateur de la PKC epsilon ;
d) déterminer le taux de la PKC epsilon dans lesdites une ou plusieurs cellules périphériques à l'étape (c) après ladite mise en contact à l'étape (c) ; dans lequel la maladie d'Alzheimer est indiquée si le taux de la PKC epsilon déterminé à l'étape (d) est supérieur au taux de la PKC epsilon déterminé à l'étape (b).

6. Procédé selon la revendication 5, dans lequel l'absence de la maladie d'Alzheimer est indiquée si ledit taux de la PKC epsilon déterminé à l'étape (d) est inférieur ou égal au taux de la PKC epsilon déterminé à l'étape (b).

7. Procédé *in vitro* pour déterminer ou surveiller la progression de la maladie d'Alzheimer, comprenant les étapes qui consistent à :
a) déterminer le taux de la PKC epsilon dans une ou plusieurs cellules périphériques d'un sujet humain ;
b) comparer le taux de la PKC epsilon dans une ou plusieurs cellules périphériques dudit sujet humain au taux de la PKC epsilon dans une ou plusieurs cellules périphériques d'un sujet de contrôle ; et
c) déterminer ou surveiller ladite progression de la maladie d'Alzheimer en se basant sur ladite comparaison à l'étape (b).

8. Procédé selon la revendication 7, dans lequel ledit taux de la PKC epsilon est un taux protéique de la PKC epsilon ou un taux d'activité de la PKC epsilon.

9. Procédé selon la revendication 7, dans lequel le taux de la PKC epsilon augmente dans une ou plusieurs cellules périphériques dudit sujet humain lorsque la progression de la maladie d'Alzheimer est inversée.

10. Procédé *in vitro* pour augmenter le taux protéique de la PKC epsilon dans une cellule, comprenant l'étape de mise en contact d'une ou de plusieurs cellules périphériques humaines avec une quantité d'un activateur de la PKC qui est efficace pour augmenter le taux protéique de la PKC epsilon dans ladite cellule périphérique par comparaison à une cellule périphérique humaine qui n'a pas été mise en contact.

11. Procédé *in vitro* pour établir le diagnostic de la maladie d'Alzheimer, comprenant les étapes qui consistent à :
b) déterminer le taux de la PKC epsilon dans une ou plusieurs cellules périphériques d'un sujet humain ;
c) mettre en contact lesdites une ou plusieurs cellules périphériques de l'étape (a) avec un peptide Aβ ;
d) déterminer le taux de la PKC epsilon dans lesdites une ou plusieurs cellules périphériques à l'étape (c) après ladite mise en contact à l'étape (c) ;
dans lequel la maladie d'Alzheimer est indiquée si le taux de la PKC epsilon déterminé à l'étape (d) n'est pas significativement différent du taux de la PKC epsilon déterminé à l'étape (b).

12. Procédé selon la revendication 11, dans lequel l'absence de la maladie d'Alzheimer est indiquée si ledit taux de la PKC epsilon déterminé à l'étape (d) est inférieur au taux de la PKC epsilon déterminé à l'étape (b).

13. Procédé *in vitro* pour identifier un composé utile pour le traitement de la maladie d'Alzheimer, comprenant :
b) la détermination du taux de la PKC epsilon dans une ou plusieurs cellules périphériques d'un sujet atteint de la maladie d'Alzheimer ;
c) la mise en contact desdites cellules périphériques avec un composé candidat ;
d) la détermination du taux de la PKC epsilon dans lesdites une ou plusieurs cellules périphériques après ladite étape (c) de mise en contact ;
dans lequel ledit composé candidat est identifié comme un composé utile pour le traitement de la maladie d'Alzheimer si le taux de la PKC epsilon déterminé à l'étape (d) est supérieur au taux de la PKC epsilon déterminé à l'étape (b).
